# EUROPEAN PATENT SPECIFICATION

(11) **EP 4 724 013 B1**
(45) Date of publication and mention of the grant of the patent: **25.11.2026**
(21) Application number: 24733530.0
(22) Date of filing: 07.06.2024
(51) Int. Cl.: A61F 2/30, A61F 2/38

(54) **TIBIAL TRAY WITH A TIBIAL TRAY SHAFT STRUCTURE AND AN ARTIFICIAL KNEE JOINT**
TIBIAPLATTFORM MIT TIBIAPLATTFORMSCHAFTSTRUKTUR UND KÜNSTLICHEM KNIEGELENK
PLATEAU TIBIAL AYANT UNE STRUCTURE DE TIGE DE PLATEAU TIBIAL ET UNE ARTICULATION ARTIFICIELLE DU GENOU

(30) Priority: 09.06.2023 EP 23178474
(43) Date of publication of application: 15.04.2026
(60) Divisional application: 26212572.7
(73) Proprietor: CeramTec GmbH, 73207 Plochingen (DE)
(72) Inventor: BISSCHOP, Bastian, 73033 Göppingen (DE); GEBERT DE UHLENBROCK, Anne, 91367 Weißenhohe (DE); HÄUSSLER, Kim Lars, 70176 Stuttgart (DE); MAIER, Dennis, 73312 Geislingen (DE); UPMANN, Carsten, 73730 Esslingen (DE)
(74) Representative: Maikowski & Ninnemann Patentanwälte Partnerschaft mbB
(86) International application number: PCT/EP2024/065766
(87) International publication number: WO 2024/251964

(56) References cited:
- EP-A2- 1 154 741
- US-A1- 2014 200 674
- US-A1- 2016 278 929
- US-A1- 2020 121 465
- US-A1- 2021 338 440

## Description

The invention relates to a tibial tray with shaft structure having the features of claim 1 and an artificial knee joint with the features of claim 17.

Joint replacements, in particular artificial knee joint replacements, have been known for quite some time. In the case of an artificial knee joint it is known that a tibial tray part is connected with the proximal end of the tibia of the patient. The connection is made through the distal part of the tibial tray, i.e. the tibial tray shaft.

A femoral part is connected with the distal end of the femur of the patient. A tibial insert is connected with the proximal surface of the tibial tray so that after the completion of the surgical procedure, the tibial insert is positioned between the distal surface of the femoral part and the proximal surface of the distal tray.

A tibial tray and a tibial insert of this kind are e.g. described in EP 3 626 209 B1, US 2021/338440 A1, EP 1 154 741 A2, US 2014/200674 A1, US 2020/121465 A1 and US 2016/278929 A1.

To secure a good cemented connection between the proximal end of the tibia and the tibial tray shaft, properly shaped geometries, such as form-fitting designs, are needed. This is important, in particular when a ceramic material is used for the tibial tray and its tibial tray shaft.

The tibial tray with the features of claim 1 addresses these issues.

The tibial tray comprises a distal surface, wherein a tibial tray shaft structure extends away from the distal surface in the distal direction by a height. The tibial tray structure forms a protrusion, which is inserted into the tibial bone.

For a good fixation of the tibial tray to the bone, the tibial tray structure comprises at least three shaft wall elements which are so arranged relative to each other that the cross-sections of the at least three shaft wall elements in a plane perpendicular to the distal direction are T-shaped, Y-shaped or X-shaped. T-shaped structure comprises three shaft wall elements, two forming the bar of the T, one forming the stem. The bar and the stem are positioned under an angle of 90°.

A Y-shaped structure also comprises three shaft wall elements, but three angles can be different from 90°. In one possible embodiment, the shaft wall elements are spaced equally apart by 120°. Alternatively, two shaft wall elements can be spaced apart by 90°, the remaining third shaft wall element is then spaced apart from the others by 135° each.

An X-shaped structure comprises four shaft wall elements. The shaft wall elements are positioned around a central axis, with angles between the shaft wall elements. It is possible, that e.g. each of the two angle sets between the shaft wall elements comprises angles of the same size (e.g. two angles of 110° each, and two angles of 70° each).

Furthermore, the area of the cross-sections decreases strictly monotonously from the distal surface in the distal direction. This means that the tibial tray structure generally tapers from a broad base into the distal direction. This facilitates an effective insertion of the tibial tray into the tibia. The tapering is continuous with sections parallel to the distal surface.

In one embodiment, outer rims of the at least three shaft wall elements converge strictly monotonously in the distal direction and towards an axis of the tibial tray structure. Again, this is a further aspect of tapering, facilitating an effective insertion of the tibial tray into the tibia.

The shape of the outer rim could, e.g., be curved with a uniform direction of curvature (e.g. an exponential shape). But the outer rims of the least three shaft wall elements have in one embodiment at least one inflection point, in particular three inflection points. This means that the direction of the curvature - not only the amount - changes at least once.

The curvature of the outer rim should not change too rapidly, so that any change of a tangent angle at an outer rim of the at least three shaft wall elements should be less than 30° over a change in distance in the distal direction of 10% of the height of the tibial tray structure, in particular any tangent angle of the outer rim of the at least three shaft wall elements within a third of the height of the tibial tray structure measured from the distal surface. The changes in the slope should be smaller in the third of the tibial shaft structure closest to the distal surface.

In a further embodiment, at least 50% of the volume of the tibial tray structure is located within a third of the height of the tibial tray structure measured from the distal surface.

At least one of the shaft wall elements can have a wall thickness between 1 and 6 mm, in particular 2 to 5 mm, for ceramic materials 1 and 5 mm, measured at the point farthest way from the axis and on the distal surface.

It is also possible that the radius at the transition from the outer rim of at least one of the at least three shaft wall elements to the sidewalls of the at least one of the at least three shaft wall element is in the range of 0,5 to 4 mm.

For efficient bonding with the bone cement, at least one of the at least three shaft wall elements comprise at least one cement holding structure, in particular at least one recess structure for retaining bone cement. Those cement holding structures can be preferably positioned on the anterior and / or posterior side close to the tibial tray.

The transition point from the outer rim into the tibial shaft structure in the distal surface is located at least 5 mm from the lateral sides and / or the posterior side of the distal surface. This ensures a suitable distance from the edge of the distal surface and makes sure that the tibial shaft structure is positioned within the substantia spongiosa and outside the substantia corticalis. The distance is measured from the end of the outer rim in a straight line to the edge of the distal surface.

In one embodiment, the tibial tray structure comprises a surface extending between at least two of the outer rims of the at least three shaft wall elements or between two side walls of the at least three shaft wall elements.

As rounding of edges can be relevant, the radius at the transition from the distal surface to at least one of the at least three shaft wall elements and / or the smooth surface can be in the range of 1 to 4 mm.

Furthermore, it is possible that the axis of the tibial tray shaft structure is located on the distal surface at one third of the distance between the anterior rim to the posterior rim, measured from the anterior rim. This means that the axis of the tibial tray shaft is located somewhat more to the anterior side than the posterior side. The axis of the tibial tray shaft is defined for X, Y, and T shaped tibial tray shaft structures, as the at least three shaft wall elements are converging towards this axis strictly monotonously. In a specific embodiment, the distance of the axis of the tibial tray shaft structure is 5 mm, 5,67 mm, 6,33 mm or 7 mm from the center of the distal surface. In combination with this or alternatively, embodiments can have tibial shaft tray structures with a height from the distal surface of 35 mm, 40 mm, 45 mm, or 55 mm. In combination with this or alternatively, the thickness of the shaft wall elements can be 3 mm, 3,75 mm, 4,25 mm or 5 mm.

The angle between the shaft wall elements is between 90° and 180° on the anterior side and between 30° to 120° on the posterior side. For example, a T-shaped tibial tray structure with three shaft wall elements would have an anterior angle of 180° and two 90° angles on the posterior side. The shapes of X- and Y-shaped tibial tray structure could vary within in the stated regions.

To ensure a good mechanical connection between the tibial tray and the tibia, the tibial tray shaft structure comprises at least one cement holding structure, in particular a hole, a groove and / or a recess without any undercut. This allows an efficient entering of cement in the cement holdings structures. In particular, the at least one cement holding structure is located within the vertical two thirds of the tibial tray shaft structure measured from the distal surface in the distal direction, in particular within the first third. This means that the at least one cement holding structure is preferably located towards the distal surface and not towards the distal end of the tibial tray shaft structure.

In a further embodiment, the tibial tray comprises least one anterior fixation element, in particular exactly one anterior fixation element, which is positioned at the anterior side of the proximal surface, the posterior side of the at least one anterior fixation element comprising a wall which in a plane parallel to the proximal surface is perpendicular to the medial plane of the proximal surface. In particular, the at least one anterior fixation element comprises at least one form-fitting element, in particular, at least one cavity in the posterior wall of the at least one anterior fixation element and / or at least one channel through the at least one anterior fixation element, for a corresponding form-fitting element of the tibial insert, the at least one form-fitting element of the at least one anterior fixation element being in particular positioned in a region in which the load transfer is at less than 70%, in particular 50% from the maximum load transfer.

The at least one anterior fixation element can comprise at least on guiding surface for a tibial insert, in particular the guiding surface having a curved and / or a plane part, in a proximal direction of the at least form-fitting element. When the tibial insert is about to be fitted together with tibial tray, a protrusion of the tibial insert contacts the guiding surface and glides along the guiding surface so that its movement during the fitting is guided and the protrusions can snap into the at least one form-fitting element. In one embodiment, the at least one guiding surface comprises a plane part or is a plane which is inclined relative to the horizontal plane by an angle in the range between 5 to 45°, in particular 30°.

In some embodiments, the tibial tray comprises a proximal surface, the proximal surface comprising at least two, in particular exactly two central fixation elements for enabling a connection with the tibial insert as a further part of an artificial knee joint, wherein the at least two central fixation elements are protrusions from the proximal surface in the proximal direction and the at least two central fixation elements are positioned symmetrically and / or parallel to a medial plane of the proximal surface, the medial plane being perpendicular to the proximal surface and intersecting the proximal surface in the middle between two most lateral points of the proximal surface. In particular, the at least two central fixation elements are symmetrically positioned relative to a frontal plane perpendicular to the medial plane, the frontal plane in particular going through the most lateral points of the proximal surface. The at least two central fixation elements can comprise a central fixation element recess for providing a better fit with the tibia insert. The at least two, or exactly two central fixation elements can be constructed as form-fitting and / or press-fitting elements.

Furthermore, it is possible that an embodiment of the tibial tray comprises at least one, in particular exactly one, posterior fixation element, positioned symmetrically to the medial plane of the proximal surface comprising a posterior wall flush with the posterior rim of the tibial tray and / or comprising an anterior wall which is essentially positioned parallel to the posterior wall of the at least one anterior fixation element. The sidewalls and / or the anterior wall of the of the at least one posterior element can in particular be tilted by an angle between 0 and 45°, in particular between 0 and 30°, more particular by an angle of 10°, relative to a plane which is perpendicular to the proximal plane and / or the sidewalls and the anterior wall of the of the at least one posterior element comprise an undercut. This enables e.g. the formation of a posterior block with inward tilted sidewalls (i.e. sidewalls with an angle greater than 0°) and sidewalls converging to the anterior side or a tongue and groove system (undercut). Such a posterior fixation element can be used as a fulcrum when attaching a tibial insert.

The tibial tray can be manufactured completely or in part from ceramic, a polymer material or a metal.

Other embodiments of a tibial tray can comprise a proximal surface, the proximal surface comprising at least two, in particular exactly two, central fixation elements for enabling a connection with a tibial insert as a further part of artificial knee joint. The central fixation elements are protrusions from the proximal surface, which can be connected as form-fitting and / or force locking with the tibial insert.

The at least two central fixation elements are designed as protrusions from the proximal surface in the proximal direction, i.e. the central fixation elements generally point away from the proximal surface in the direction of the tibial insert.

The at least two central fixation elements are positioned symmetrically and / or parallel to a medial plane of the proximal surface, the medial plane being perpendicular to the proximal surface and intersecting the proximal surface in the middle between two most lateral points of the proximal surface. The medial plane is dividing the proximal surface into two lateral halves. The at least two central fixation elements are oriented symmetrically and / or parallel to the medial plane. In a further embodiment the at least two fixation elements are oriented asymmetrically relative to the medial plane. This can imply different angles relative to the medial plane and / or different distances from the medial plane.

The at least two central fixation elements are configured as cylindrical protrusions or linear protrusions, in particular designed for a press-fit and / or form-fit. Cylindrical means that the protrusion has e.g. a circular, elliptical cross-section. Linear means that the protrusion extends linearly on the proximal surface. At least one of the central fixation elements can e.g. have a linear or cylindrical shape.

In one embodiment, the at least two central fixation elements are symmetrically positioned relative to a frontal plane perpendicular to the medial plane. The frontal plane is dividing the proximal surface into an anterior and a posterior part. In particular, it is possible that the frontal plane is going through the most lateral points of the proximal surface.

For a better insertion of the tibial insert and for a better fixation in vivo, the posterior wall of the at least one anterior fixation element and / or the anterior wall of the at least one posterior fixation element can comprise an undercut.

In one embodiment, the height of the at least two central fixation elements, the at least one anterior fixation element and / or the at least one posterior fixation element extending from the proximal surface is in the range between 1 mm and 6 mm, in particular between 3 mm and 4 mm, 5 mm and / or wherein the anterior and / or posterior ends of the at least two central fixation elements are rounded. In particular, the height of the at least two central fixation elements, the height of the at least one anterior fixation element and / or the height of the at least one posterior fixation element above the proximal surface is constant or varies by maximally 10% from the minimal height. These features are instrumental for providing a secure connection with the tibial insert and / or allow an effective assembly.

The issues are also addressed by a tibial insert designed or configured to match the tibial tray as claimed.

To ensure a good connection with the tibial tray, the tibial insert can comprise one recess in the distal surface for the matching of at least one posterior fixation element, the recess comprising at least one stress relief notch at the junction of two walls of the recess. This prevents the build-up of mechanical stress sharp corners where two walls of the recess meet.

The secure connection can be enabled by at least one form-fitting element of the tibial insert for engaging the at least one form-fitting element of the at least one anterior fixation element of the tibial tray. The connection can also be made with a press-fitting connection or a combination.

The issues are also addressed by an artificial knee joint comprising a claimed tibial tray and a claimed tibial insert, wherein there is a light press-fit connection between the tibial insert and the at least two central fixation elements, the at least one anterior fixation element and the at least one posterior fixation element, in particular having a press-fit in the range of 0 to 450 µm, in particular in the range of 0 to 250 µm.

In a further embodiment of an artificial knee joint, there is at least one press-fitting region between the posterior fixation element and the tibial insert, in particular created by an angular mismatch between two walls. This allows for an improved assembly of the joint and a reduction of micromovements in the posterior parts of the joint.

Embodiments of the invention are shown in the figures,
- Fig. 1: showing a perspective view of an artificial knee joint;
- Fig. 2: showing a perspective view onto the distal surface of an embodiment of a tibial tray with a tibial tray structure with three shaft wall elements (Y-structure);
- Fig. 3: showing a perspective view onto the distal surface of an embodiment of a tibial tray with a tibial tray structure with four shaft wall elements (X-structure);
- Fig. 4: showing a review onto the distal surface of a further embodiment of a tibial tray with a tibial tray structure with three shaft wall elements (T-structure);
- Fig. 5: showing the structure of Fig. 4 with a cross-sectional plane in the proximal third of the tibial stray structure (T-structure);
- Fig. 6: showing a perspective view onto the distal surface of the embodiment shown in Fig. 4 and 5 (T-structure);
- Fig. 6A: showing a view of the distal surface of an embodiment with indications of radii of the perimeter;
- Fig. 7: showing a perspective view from the side of an embodiment of a tibial tray with a tibial tray structure with three shaft wall elements (T-structure) and a recess structure for bone cement;
- Fig. 8: showing a perspective view onto the distal surface of an embodiment of a tibial tray with a tibial tray structure with three shaft wall elements (T-structure);
- Fig. 9: showing a perspective view of an embodiment of a tibial tray;
- Fig. 9A: showing the same view of Fig. 9 highlighting reference frames;
- Fig. 9B: showing a variation of the embodiment in Fig. 9;
- Fig. 10A: showing a view of the proximal surface of an embodiment of a tibial tray;
- Fig. 10B: showing a view of the distal surface of a tibial insert matching the tibial tray shown in Fig. 10A;
- Fig. 11: showing a view of a proximal surface of the embodiment shown in Fig. 10A indicating interference regions;
- Fig. 12A: showing the insertion of a tibial insert to a tibial tray from a perspective view;
- Fig. 12B: showing the of the tibial insert to the tibial tray in Fig. 12A in a sectional view;
- Fig. 13A: showing a detailed frontal view of a first embodiment of the tibial insert;
- Fig. 13B: showing a detailed frontal view of a second embodiment of the tibial insert;
- Fig. 14: showing a perspective view of a variation of the embodiment shown in Fig. 9;
- Fig. 15A: a perspective view of the distal surface with a view from the anterior side on the tibial shaft structure;
- Fig. 15B: a perspective view of the distal surface with a view from the posterior side on the tibial shaft structure;
- Fig. 15C: a view in proximal direction on the distal surface;
- Fig. 16A: a perspective view from the posterior side of the proximal surface of the tibial tray with a first embodiment of central fixation elements;
- Fig. 16B: a perspective view from the anterior side of the proximal surface of the tibial tray with the first embodiment of central fixation elements;
- Fig. 17: a perspective view from the posterior side of the proximal surface of the tibial tray with a second embodiment of central fixation elements;
- Fig. 18A: a cross-sectional view through an embodiment of the tibial tray in the anterior direction;
- Fig. 18B: showing a cross-sectional view through an embodiment of the tibial tray in the posterior direction;
- Fig. 19: showing a schematic representation of line elements to generate a smooth outer rim for wall elements;
- Fig. 20A: showing a perspective view of the anterior side of an embodiment of a tibial tray with a cement holding structure;
- Fig. 20B: showing a perspective view of the posterior side of the embodiment shown in Fig. 20A with a cement holding structure;
- Fig. 21A: showing a perspective view of the anterior side of an embodiment with an illustration of the geometry at wall elements of tibial tray shaft structure;
- Fig. 21B: showing a perspective view of the posterior side of the embodiment shown in Fig. 21A;
- Fig. 22: showing a perspective view onto the proximal surface of a further embodiment of tibial tray with guiding surfaces on the anterior fixation element;
- Fig. 23: showing a detail of the connection between the posterior fixation elements in one embodiment.

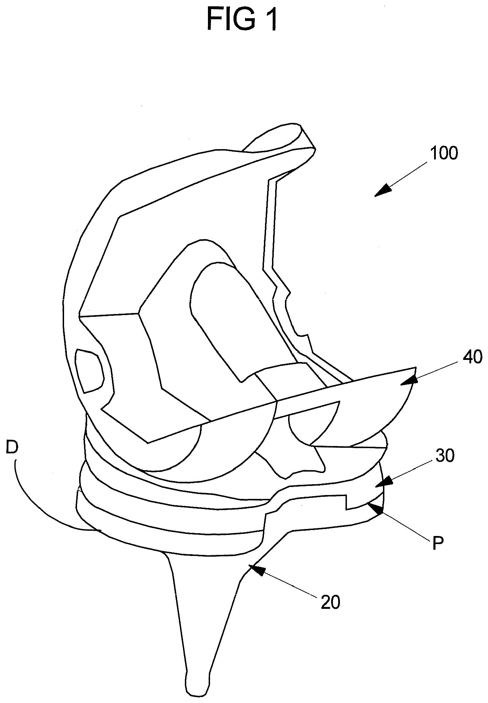

In Fig. 1, an artificial ceramic knee joint 100 is shown in a perspective view from a posterior position. In Fig. 1, a femoral part 40, a tibial insert 30 and a tibial tray 20 of the metal-free artificial knee joint are shown. Alternative embodiments are not metal-free.

Here the complete artificial art knee joint 100 is metal-free, e.g. a combination of ceramic and polymeric materials. The tibial tray 20 can, e.g., be made from ceramic and the tibial insert 30 can be made from a polymeric material.

At the proximal position of the artificial knee joint, a femoral part 40 is shown with two condyles fitting into matching grooves in the proximal surface of a tibial insert 30 at the distal end of the femoral part 40.

The tibial insert 30 is connected with a tibial tray 20, the distal surface D of the tibial insert 30 facing the proximal surface P of the tibial tray 20.

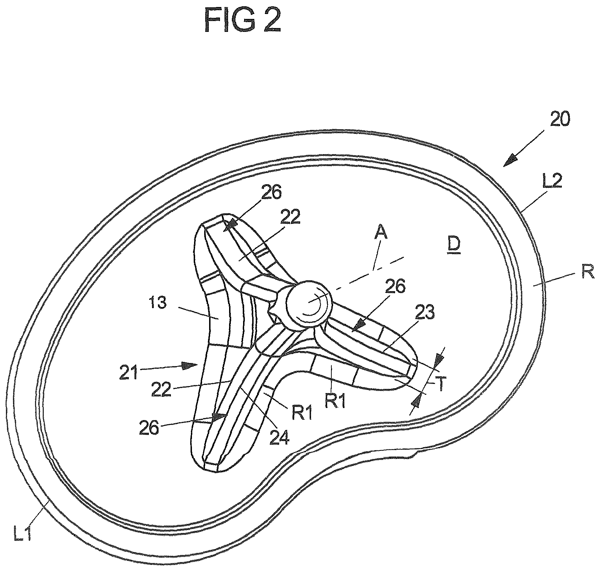

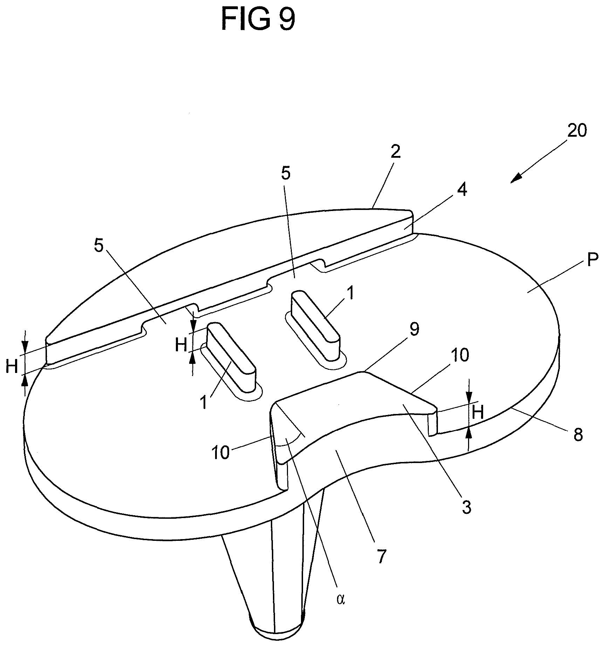

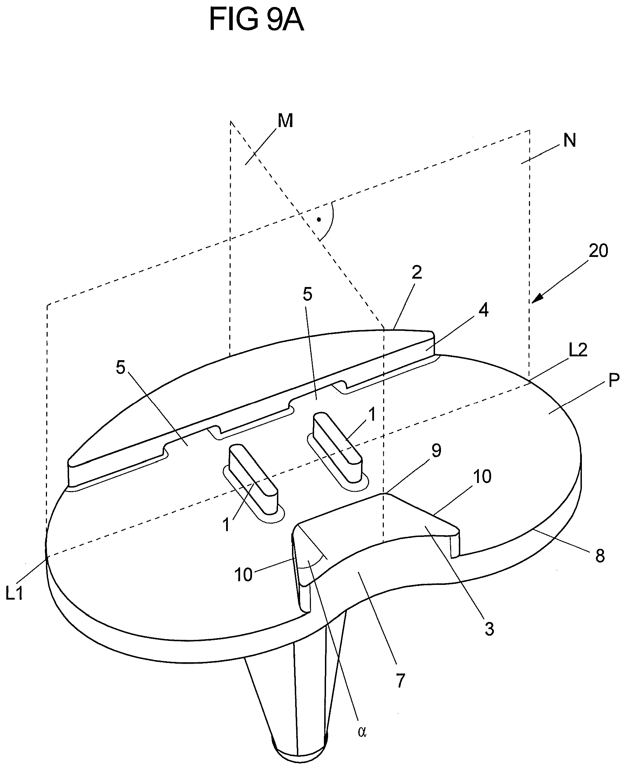

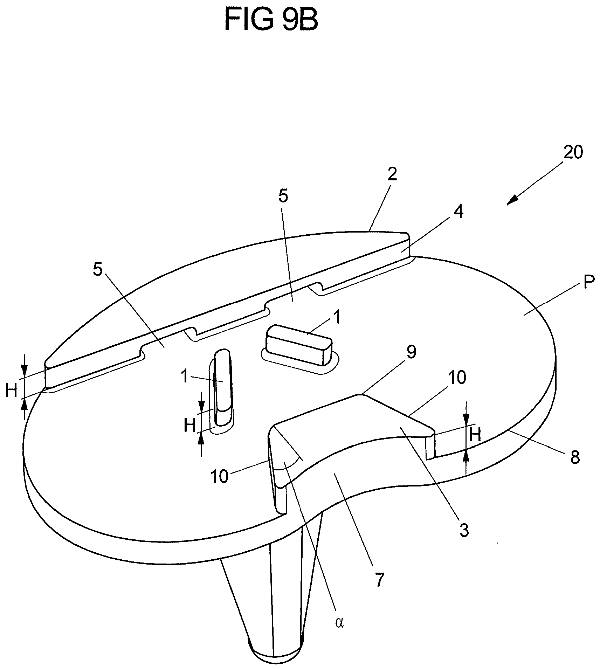

In connection with Fig. 2 to 8, embodiments of a tibial tray 20 are described. In Fig. 9 to 14, embodiments of a tibial tray 10 and a tibial insert 30 are described which can be used in connection within the embodiments of the tibial tray 20 described herein.

In Fig. 2, a perspective view of the distal side of an embodiment of a tibial tray 20 is shown. The shape of the distal surface D is roughly shaped as the proximal side of the tibial tray 20 (see e.g. Fig 9 to 15). A tibial tray shaft structure 21 is located in the center of the distal surface D, i.e. in the middle of the lateral width between points L1 and L2. In the embodiment shown, the tibial shaft structure 21 is essentially Y-shaped (i.e. is referring to a cross-section in a plane parallel to the distal surface D) with an axis A in the center. The tibial tray structure 21 comprises three shaft wall elements 22, 23, 24 that are placed around the axis A with angles of 120° between them. One shaft wall element 22 of the Y is oriented to the anterior side of the tibial tray 20, the two other shaft wall elements 23, 24 are oriented to the posterior side of the tibial tray 20.

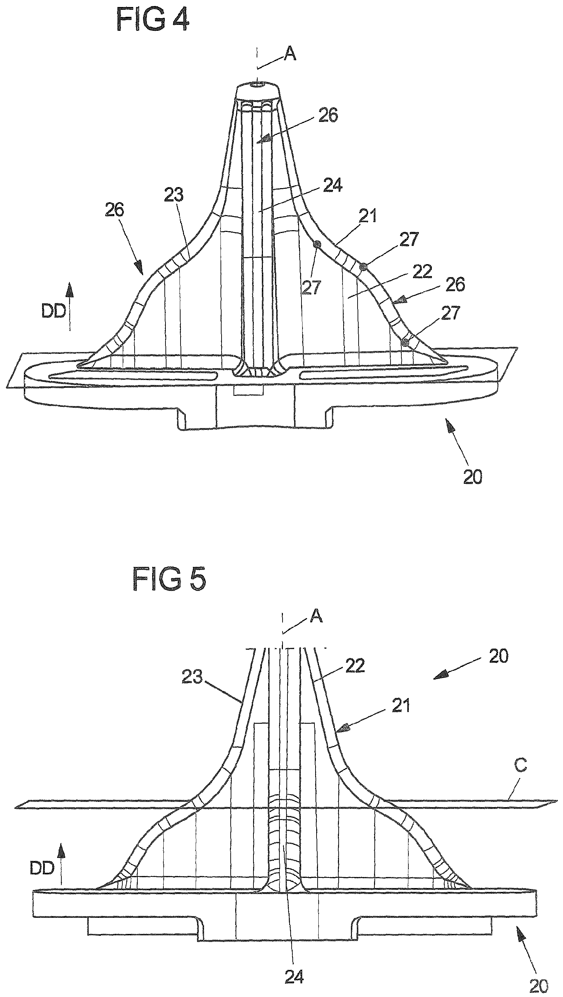

The tibial tray structure 21 extends away from the distal surface D into a distal direction DD (as indicated e.g. in Fig. 5) that in Fig. 2 is essentially pointing out of the plane of the figure.

At the rim of the distal surface D an elevated rim R is located, extending away from the distal surface D. The width in the plane parallel to the distal surface D is between 1 to 4 mm.

The three shaft wall elements 22, 23, 24 of the tibial tray shaft structure 21 each comprise an outer rim 26. The outer rims 26 are thin faces of the shaft wall elements 22, 23, 24. The thickness T of the shaft wall elements 22, 23, 24 measured at the endpoints (i.e. the points farthest away from the axis A on the distal surface D) is between 2 and 5 mm.

The outer rims 26 converge from their broad base at the distal surface D towards the axis A in the distal direction. The convergence is strictly monotonous, i.e. there is no section of the outer rims 26 being parallel to the distal surface D. In the embodiment shown in Fig. 2, the slope of the outer rims 26 is increasing from the base, i.e. the distal surface D, towards the axis. The change of the slope is smooth, without kinks.

In the space subtended between the shaft wall elements 22, 23, 24, the outer rims 26 are connected by a smooth surface S, here an inward (i.e. towards the axis A) curved surface. The surface 13 itself has no kinks or edges to enable a good insertion into the tibia. The transition from the smooth surface 13 to the outer rim 26 is also smooth, i.e. without kinks or edges. In the embodiment shown, the radius R2 of this transition is between 1 and 4 mm, in particular depending on the size of the tibial tray structure 21.

The transition from the distal surface D into the shaft wall elements 22, 23, 24 and / or the smooth wall 13 is rounded with the radius R1 being in the range of 1 to 4 mm.

As will also be shown in the context of Fig. 5 in more detail, the cross-sectional area of the tibial shaft structure 21 in a plane C parallel to the distal surface D is greatest at the base at the distal surface D. The cross-sectional area is getting smaller (in particular strictly monotonous smaller) as the tibial shaft structure 21 extends into the distal direction DD.

The base plate with the distal surface D has a thickness in the range of 2 mm to 6 mm, in particular 4,5 mm.

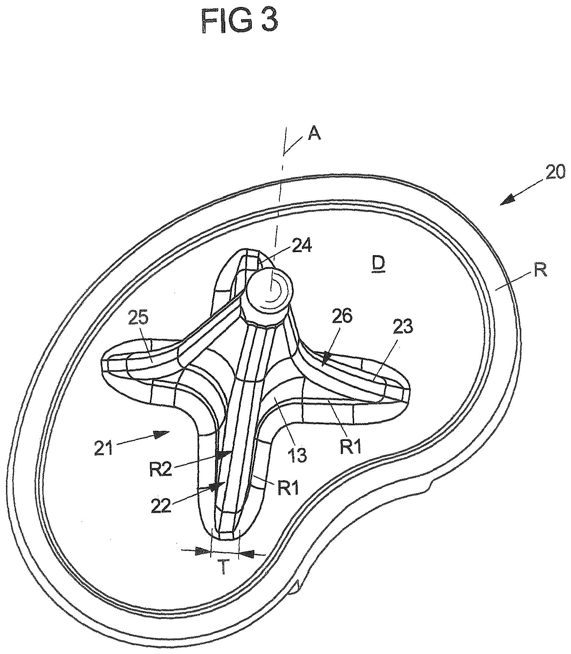

In Fig. 3, an embodiment of a tibial tray 20 is shown, which comprises four shaft wall elements 22, 23, 24, 25 in an X-shaped pattern. Two of the shaft wall elements 24, 25 are oriented towards the anterior side of the tibial tray 20, two of the shaft wall elements 22, 23 are oriented towards the posterior side.

Other than that, the geometry of the outer rims 26, the smooth surface 13 in the angled space between the shaft wall elements 22, 23, 24, 25 and in particular the convergence of the outer rims 26 in the distal direction D towards the axis A are analog to the embodiment shown in connection with Fig. 2, so that reference to the description can be made.

The embodiment shown in Fig. 4 in a perspective view is a further variation of the embodiment shown in Fig. 2 and 3. Hence, the above given description is applicable as well.

Here, the tibial tray structure 21 has a T-shaped cross-section in a plane parallel to the distal surface D. The shaft wall elements 22, 24 forming the bar of the T are positioned essentially parallel to anterior side, one shaft wall element 25 forming the stem of the T is directed perpendicularly to the posterior side of the tibial tray 20.

Again, the three shaft wall elements 22, 23, 24 of the tibial tray structure 21 have cross-sectional areas, which converge strictly monotonously towards in the distal direction DD towards the axis A. The outer rims 22, 23, 24 have slopes (measured against the distal plane) which are always greater than 0°, i.e. the outer rims have no plateaus. But the outer rims 26 have three inflection points 27 in which there is a change in curvature.

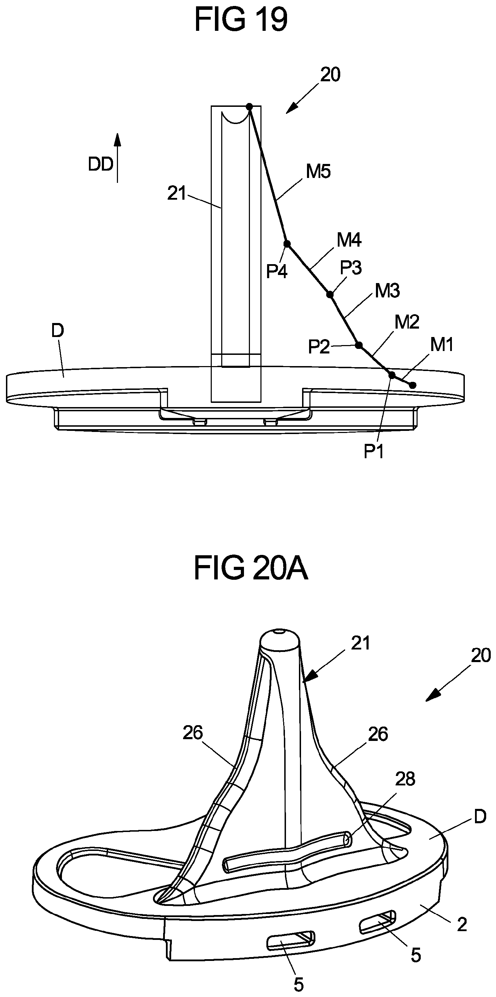

Any change of a tangent angle over a 10% height distance of the tibial tray structure that means that there are no sharp bends in the outer rim 26. The changes in the slope are less in the first third of the height of the tibial tray structure 21 measured from the distal surface. A further definition of the outer rim 26 is described in connection with Fig. 19.

Fig. 5 shows the embodiment of Fig. 4 in a rear view. Here, a sectional plane C parallel to the distal surface D is shown. The sectional plane C separates the lower third of the tibial tray structure 21 from the upper two thirds. For a good mechanical load distribution, at least 50% of the volume of the shaft wall elements 22, 23, 24 are between this sectional plane C and the distal surface D, i.e. in the third closest to the distal surface D. This is also the case for the other embodiments shown in Fig. 2 and 3.

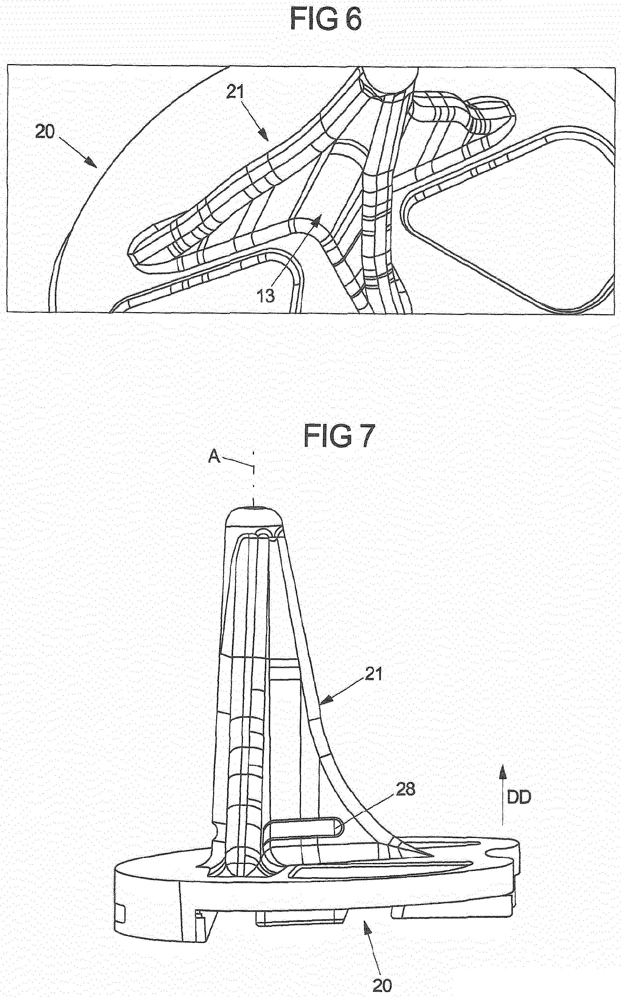

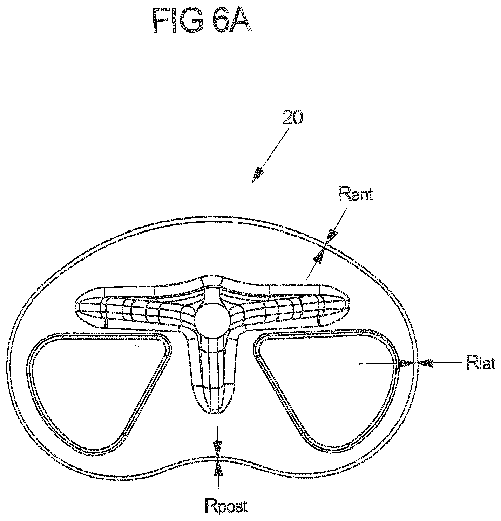

Fig. 6 shows the same embodiment as the one shown in Fig. 4 and 5, i.e. a tibial tray structure 21 with a T-structure. In this view, the shape of the outer rim 26 and the rounded transitions from the distal surface into the tibial stray structure 21 which can be seen clearly in the figure.

In Fig. 6A, some geometry of the perimeter of the tibial tray 20 is described. The anterior radius Rant is between 30 mm and 60 mm. The lateral radius Rlat is between 15 mm and 30 mm and the posterior radius Rpost is between 10 mm and 20 mm, in particular 16 mm.

Fig. 7 basically shows the embodiment of Fig. 4 to 6 in a side view, so that the above description is applicable. In addition to the embodiments described, this embodiment comprises one or more anterior and / or posterior cement holding structures, here recess structures 28 in the tibial tray structure 21. When implanting the tibial tray 20 into the tibia bone cement can enter the kind of undercut for better fixation. The recess structure 28 is shaped like a groove essentially parallel to the distal surface in this embodiment. Other shapes are possible in principle. One aspect here is that cement holding structure 28 should be oriented in a way that the bone cement is not easily removed when implanting the tibial tray 20.

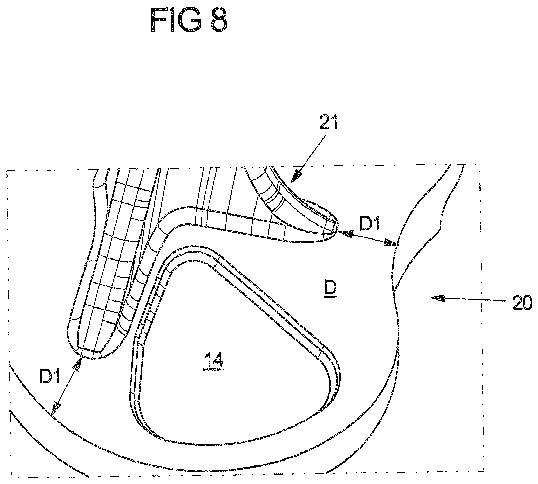

In Fig. 8, a variant of the embodiment shown in Fig. 4 to 7 is depicted, i.e. a T-structure as a tibial tray structure 21. The distal surface D comprises a recess 14. Furthermore, it is indicated that transition point from the outer rim 26 of the tibial shaft structure 21 in the distal surface D is located at least 5 mm (D1) from the lateral sides and / or the posterior side of the distal surface D. The middle plane of the shaft wall elements 22, 23 are extended to the lateral sides / and or the posterior side of the distal surface D, defining the distances D1. This allows a good placement of the tibial tray structure 21 in the bone.

In the following, embodiments of the tibial tray 20 and the tibial insert 30 are described, in particular features of the proximal surface P of the tibial tray 20 and the matching distal surface D of the tibial insert 30.

Fig. 9, 9A show a perspective view onto the proximal surface P of the tibial tray 20. In Fig. 9A the tibial tray is shown for reducing complexity without the reference numbers but with reference planes M, N which are used in the following to define the locations of features on the tibial tray 20.

Referring to Fig. 9A, a medial plane M is perpendicular to the planar proximal surface P and intersecting the proximal surface P in the middle between to most lateral points L1, L2 of the proximal surface P. A frontal plane N is perpendicular to the medial plane M.

In one embodiment of the tibial tray 20, the dimensions are grouped in different sizes. There are eight different sizes for the distance between the points L1 and L2. This is used to allow for artificial knee joints 100 for differently sized patients. The smallest distance between L1 and L2 is 60 mm. The other seven sizes have distances of 64 mm, 68 mm, 72 mm, 76 mm, 80 mm, 84 mm and 88 mm (i.e. using an increment of 4 mm). Other embodiments can use different absolute sizes and / or different increments, the increments do not have to be identical.

In the extent of the proximal surface P in the direction perpendicular to the distance between L1 and L2, the corresponding sizes are 38,7 mm, 41,3 mm, 43,9 mm, 46,5 mm, 49,0 mm, 51,6 mm, 54,2 mm and 56,8 mm. Other embodiments can use different absolute sizes and / or different increments, again, the increments do not have to be identical.

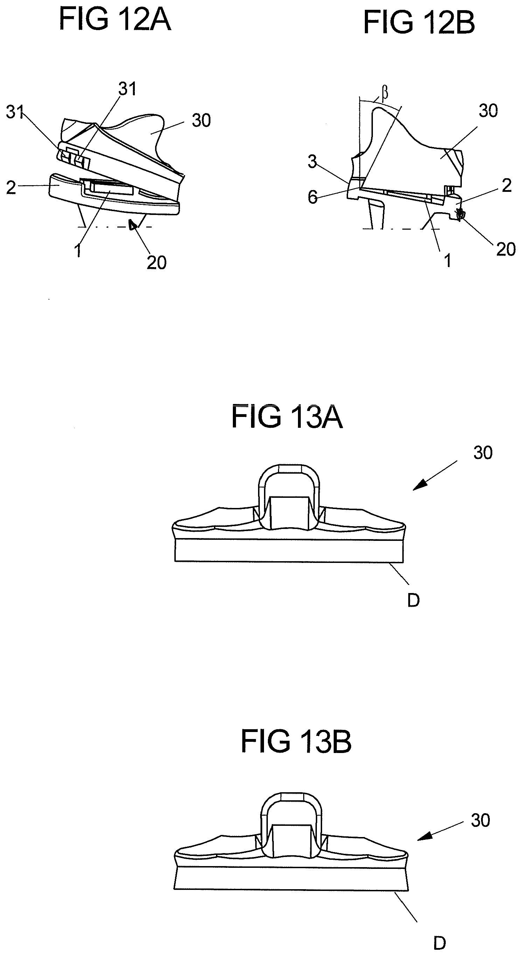

The proximal surface P of the tibial tray 20 is structured so that a corresponding tibial insert 30 (see e.g., Fig. 1, 13) can be securely fastened to the tibial tray 20 (see Fig. 12A, 12B). The planar proximal surface P itself is polished with a mean roughness of Ra=1 µm, in particular Ra=0,1 µm and an evenness of 0,1. In other embodiments, the mean roughness is at less than 5 µm, in particular at less than 2 µm.

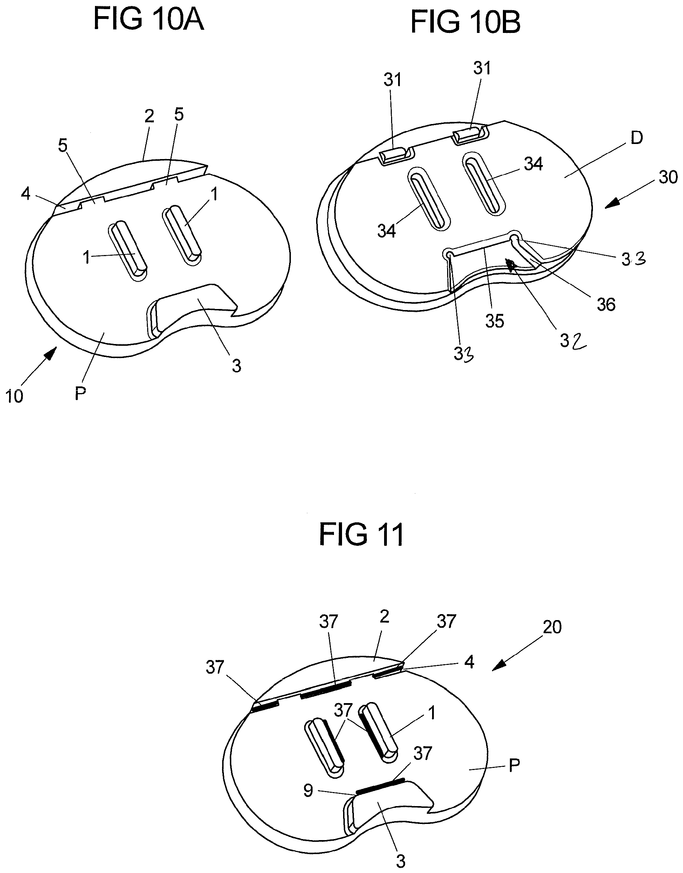

In the following, different aspects of the structures 1, 2, 3 on the planar proximal surface P are described. The structures provide a symmetric, flat plateau with island-like fixation elements 1, 2, 3 for the tibial insert 30 connection using a press-fit or interference-fit (see Fig. 11).

In the embodiment shown in Fig. 9, the proximal surface P comprises two central fixation elements 1. As will be described below, those two central fixation elements enable a secure connection with the tibial insert (see Fig. 10B) as a further part of the artificial knee joint 100.

In the embodiment shown, the two central fixation elements 1 are linear protrusions, protruding away from the proximal surface P in the proximal direction, so that they can match corresponding notches (or grooves) 34 in the tibial insert 30 (see Fig. 10B). In other embodiments, the central fixation elements 1 could have a cylindrical shape. Other shapes and different positions of the central fixation elements 1 will be shown below.

The two central fixation elements 1 are positioned symmetrically and parallel to the medial plane M.

In an embodiment shown in Fig. 9B, the two central fixation elements 1 are still symmetric to the medial plane M, but not quite parallel as they are angled by less than 5° relative to the median plane M, so that the two linear fixation elements 1 show some convergence towards the anterior side.

In other embodiments, it is also possible that the two central fixation elements 1 are asymmetric to the median plane M but parallel to each other.

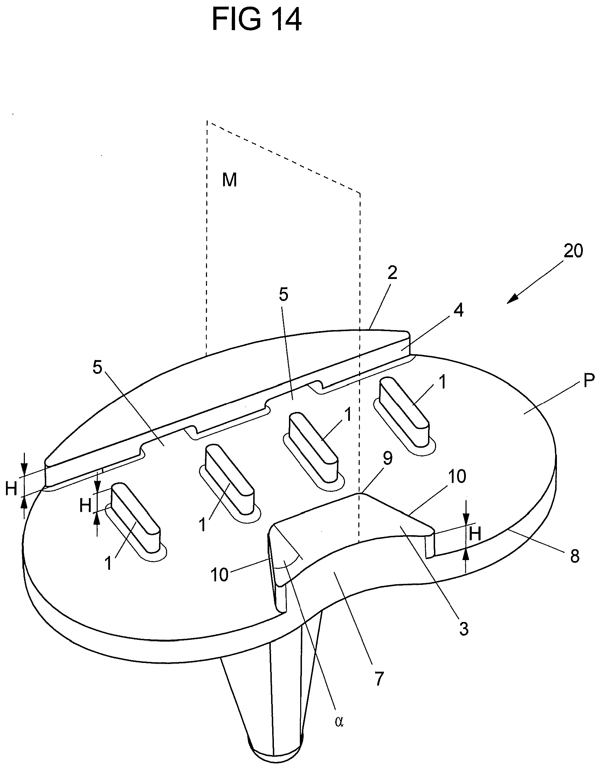

As it is shown in Fig. 14, in a different embodiment, more than two central fixation elements 1 can be used. Otherwise, the embodiment of Fig. 14 is comparable to the one described in connection with Fig. 9.

In the embodiment shown in Fig. 9, the two central fixation elements 1 are symmetrically positioned relative to the frontal plane N perpendicular to the medial plane M. In this embodiment, but not necessarily, the frontal plane N is going through the most lateral points L1, L2 of the proximal surface P.

The two central fixation elements 1 have the form of a parallelepiped, the anterior and the posterior ends of the central fixation elements 1 are rounded.

The two central fixation elements 1 have a height H extending in the embodiment shown 3,8 mm from the proximal surface P of the tibial tray 20. In other artificial knee joints 100, the height H can be up to 6 mm. In the embodiment shown, the height H of the central fixation elements 1 is constant. In other embodiments, the height H can vary relative to the proximal surface P. In other embodiments, the height H of the at least two central fixation elements 1 over the proximal surface varies by maximal 10% from the minimal height.

In the embodiment shown in Fig. 9, the distance between the two central fixation elements 1 is 10,5 mm as measured between the interior walls and 15,5 mm as measured between the exterior walls. As the two central fixation elements 1 are parallel to each other, the width of each of the central fixation elements is 2,5 mm. The distance between the central fixation elements is chosen here to be as large as possible to provide a large torque and to prevent a shearing off of polyethylene material from the tibial insert 30. If the distance between the two central fixation elements 1 is always the same, tibial inserts 30 of different sizes can be used in assembling the artificial knee joint 100.

The orientation of the central fixation element 1 on the proximal surface P enables a linear guiding of the tibial insert 30 during the assembly (see Fig. 12A, 12B), so that the central fixation elements 1 could also be termed as central guide rails. Furthermore, the central fixation elements 1 allow for an increased stability against shear forces in the artificial knee joint.

The proximal surface P also comprises an anterior fixation element 2 that is located at the anterior rim of the proximal surface P. The posterior side of the anterior fixation element 2 comprises a posterior wall 4 that is positioned perpendicular to the medial plane M of the proximal surface P. Therefore, the posterior wall 4 is also essentially perpendicular to the two linear central fixation elements 1. The anterior fixation element 2 in this embodiment has the same height H as the central fixation element 1. In other embodiments the heights of the anterior fixation element 2 and the central fixation elements 1 can be different.

The anterior fixation element 2 comprises two cavities 5, each in the form of a channel (i.e. the channel being open at both ends) with an essentially rectangular cross-section. As will be shown in connection with Fig. 12A, 12B, those cavities are form-fitting elements 5 cooperating with matching form-fitting elements 31 on the tibial insert 30. The form-fitting elements 5 in the anterior fixation element are positioned symmetrically relative to the medial plane M.

In other embodiments, the anterior fixation element 2 comprises only one or more than two form-fitting elements 5. The cross-section of the cavities of the form-fitting elements 5 does not have to be rectangular, as e.g. round or polygonal cross-sections can also be used.

In the embodiment of Fig. 9, the anterior fixation element 2 is one continuous element at the anterior rim of the tibial tray 20. In other embodiments, the anterior fixation element 2 can be divided into more than one part, e.g. two elements, each of them comprising a form-fitting element 5.

The two form-fitting elements 5 in the anterior fixation element 2 are positioned in a region in which the mechanical load transfer is at less than 50% from the maximum load transfer. This is due to the fact that the channel-like form-fitting elements 5 somewhat weaken the structure of the anterior fixation element 1.

A further structure on the proximal surface P is a posterior fixation element 3, which is positioned at the posterior rim of the proximal surface P. The posterior fixation element 3 is positioned symmetrically relative to the medial plane M and comprises a posterior wall 7 flush with the posterior rim 8 of the tibial tray 20. The posterior wall 7 is following the curved shape of the posterior rim 8 of the tibial tray 20. The cross-section of the anterior fixation element 3 in a plane parallel to the proximal surface P is symmetric to the medial plane M. The posterior fixation element 3 in this embodiment has the same height H as the central fixation element 1. In other embodiments the heights of the posterior fixation element 3 and the central fixation elements 1 can be different.

The embodiment shown in Fig. 9 also comprises an anterior wall 9 of the posterior fixation element 3 and a posterior wall 4 of the anterior fixation element 2. The respective rims of those walls 4, 9 at the proximal surfaces of the posterior element 3 and the anterior fixation element 2 are parallel to each other. If the walls, 4, 9 would have no undercut (i.e. the walls 4, 9 are not tilted), the walls 4, 9 themselves would be parallel. Therefore, the two central fixation elements 1 are oriented perpendicular to both the posterior wall 4 of the anterior fixation element 2 and the anterior wall 9 of the posterior fixation element 3.

The posterior fixation element 3 also comprises two sidewalls 10 that are angled relative to the anterior wall 9 of the posterior fixation element 3. The angle α between the two sidewalls 10 and the anterior wall 9 in a plane parallel to the proximal plane P is in the range between 0° and 22°, in particular in the range between 15° and 22°. Therefore, the two sidewalls 10 are oriented convergent in the anterior direction. The overall horizontal shape of the posterior fixation element 3 is roughly trapezoidal, with the long side of the trapezoid being curved.

The two sidewalls 10 of the posterior fixation element 3 are tilted inwards to form an undercut by an angle between 1 and 45°, in particular between 20 and 30° relative to a plane which is perpendicular to the proximal plane P. Therefore, the top surface of the posterior fixation element 3 is slightly larger than the base cross-section in the plane of the proximal surface.

In the embodiment described herein, and best shown in Fig. 12B, the anterior wall 9 of the posterior fixation element 3 comprises an undercut 6, the undercut 6 forming an angle β between 1° and 45°, in particular 30° with a plane perpendicular to the proximal surface P. In other embodiments, the undercut 6 can have - at least in parts - a curved shape. It is in principle also possible that the posterior wall 4 of the anterior fixation element 2 comprises an undercut 6.

In the embodiments discussed so far, the height H of the fixation elements 1, 2, 3 above the proximal surface P was identical. That does not have to be the case in all embodiments.

The matching tibial tray 20 and tibial insert 30 are shown side by side in Fig. 10A, 10B. The embodiment of the tibial tray 20 in Fig. 10A is already described in Fig. 9, so reference can be made to the respective description. The distal surface D of the tibial insert 30 is shown in Fig. 10B. The comparison of the two parts 20, 30 shows that the tibial insert 30 can be connected with the tibial tray 20 so that the first fixation elements 1 fit into matching notches 34 in the tibial insert 30.

The tibial insert 30 comprises a recess 32 (see Fig. 10B) in the distal surface D for the matching posterior fixation element 3 of the tibial tray 20. The recess 32 is open towards the posterior side so that it only has an anterior wall 35 and two sidewalls 36.

The sidewalls 36 of the recess 32 are angled to match the angled sidewalls 10 of the posterior fixation element 3. In the corners where the anterior wall 35 and the two sidewalls 36 of the recess 32 meet, stress relief notches 33 are located at the junction of the two walls 35, 36.

At the anterior side of the tibial tray insert 30, two form-fitting elements 31 are positioned which can be inserted into the two cavities of the form-fitting elements 5 in the anterior fixation element 2 (see Fig. 9).

The notches 34 and the recess 32 of the tibial tray 30 also form some form-fitting connection with the matching structures, i.e. the central fixation elements 1 and the posterior fixation element 3 of the tibial tray 20. But the main function of the structures 32, 34 is an assistance during the assembly which will be described below in connection with Fig. 12A, 12B.

The connection between the tibial tray 20 and the tibial insert 30 is primarily effected by a press-fit connection between the tibial insert 30 and the at least two central fixation elements 1, the at least one anterior fixation element 2 and the at least one posterior fixation element 3, in particular having a press-fit in the range of 0 to 450 µm, more in particular in the range of 0 to 250 µm in the anterior-posterior direction. In Fig. 11, the surfaces 37 for the press-fit at the posterior wall 4 of the anterior fixation element 2, at the inside of the central fixation elements 1 and the anterior wall 9 of the posterior fixation element 9 are highlighted. It is possible that all press-fit connections are dimensioned identically, but that does not have to be the case for all embodiments. For example, the press-fit between the posterior wall 4 of the anterior fixation element 2 and the anterior wall 9 of the posterior fixation element 3 is in the range of 50 to 150 µm and / or the press-fit between the central fixation elements 1 is in the range of 50 and 100 µm. The lateral surfaces of the recess 32 provide an interference-fit.

In Fig. 12A and 12B, the assembly of embodiments of a tibial insert 30 and a tibial tray is shown in different views.

Fig. 12A shows a perspective view from the front with the tibial insert 30 partially connected with the tibial tray 20 underneath. Fig. 12B shows essentially the same relative position of the tibial tray 20 and the tibial insert 30 but in a sectional view, with the anterior fixation element 2 on the right-hand side.

The tibial insert 30 is first connected with the posterior fixation element 3 (see Fig. 9, 10A, 10B) which is used as some kind of fulcrum in the snapping into place. In the view of Fig. 12B, the undercut 6 in the anterior wall 9 with an angle of about 15° is shown which allows a secure positioning of the tibial insert 30. At the anterior side (see Fig. 12A), the two form-fitting elements 31 are shown which snap into the cavities of the form-fitting element 5 (not seen in Fig. 12A).

In Fig. 13A, a first embodiment of a tibial insert 30 is shown in a frontal view. In this embodiment, the shape of the distal surface D is not fitted to the footprint, i.e. the outer rim of the tibial insert 30 is essentially a slightly inclined wall. The inclination can be between 0 and 10°. In case of an inclination of 0° the wall is straight.

In Fig. 13B, a second embodiment of a tibial insert 30 is also shown in a frontal view. Here, the shape of the distal surface D is fitted to the footprint, i.e. the outer wall is lightly tilted outwards.

Even though, the above-described ceramic knee joint 100 with a metal-free, in particular ceramic, tibial tray 20 is a preferred embodiment, it is possible that the tibial tray 20 or the complete knee joint 100 are made from metal or from polymer material or that they comprise these materials.

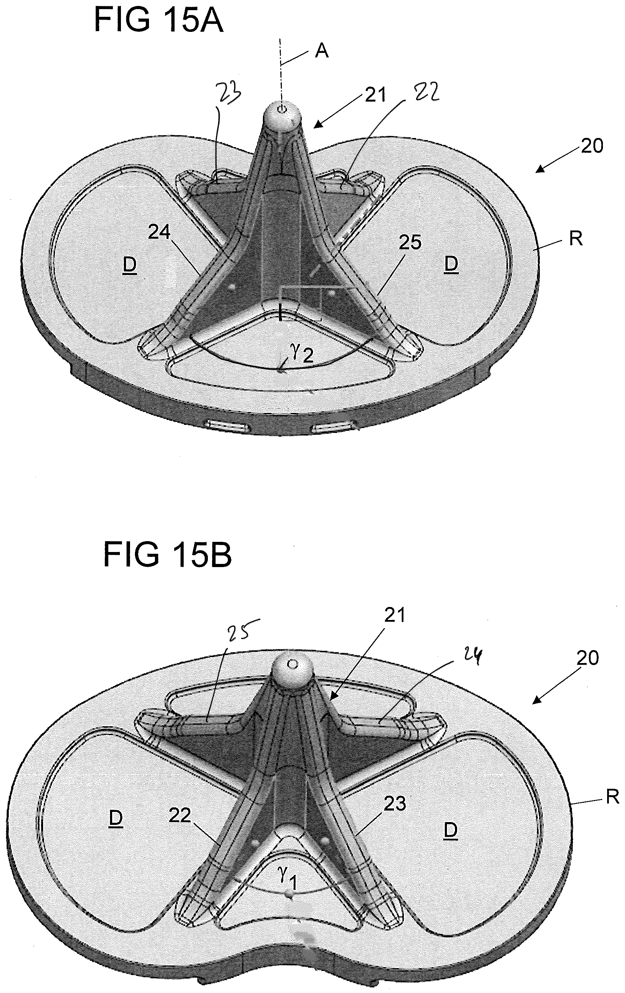

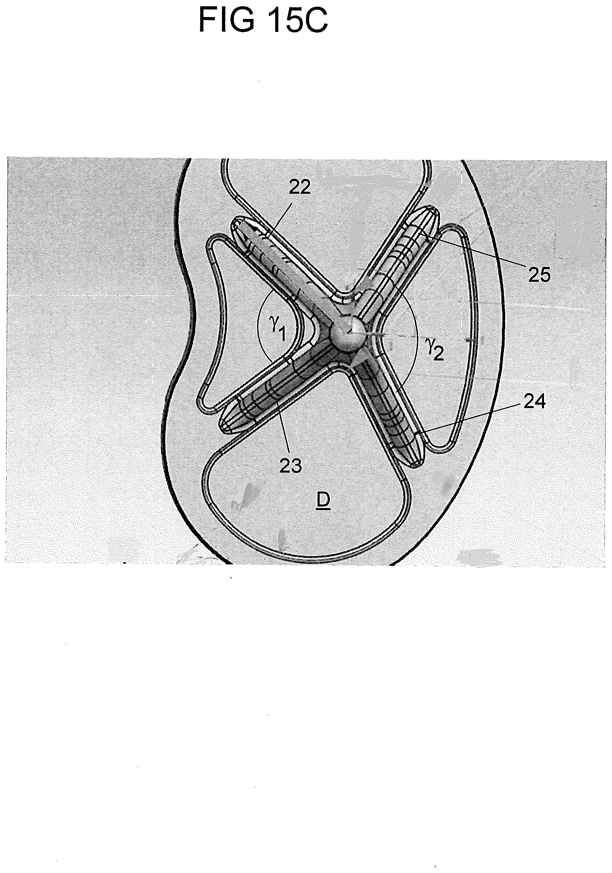

Fig. 15A, 15B and Fig. 15C show the distal surface D of the tibial tray 20. Fig. 15A shows a view from the anterior side, Fig. 15B shows a view from the posterior side. Fig. 15C shows a top view of the distal surface D.

The tibial tray structure 21 is essentially an X-shaped structure, in which the angle γ₁ between the first and second shaft wall elements 22, 23 differs from angle γ₂ between the third and fourth shaft wall elements 24, 25. The angle γ₁ between the first and second shaft wall elements 22, 23 facing the posterior side is smaller than the angle γ₂ between the third and fourth haft wall elements 24, 25. In the embodiment shown, the angle γ₁ between the first and second shaft wall elements 22, 23 is less than 90°, i.e. 80° (see Fig. 15B); the angle γ₂ between the third and fourth shaft wall elements 22, 23 is larger than 90°, i.e. 120°. In one embodiment the angles γ₁, γ₂ are 110° and 70° respectively.

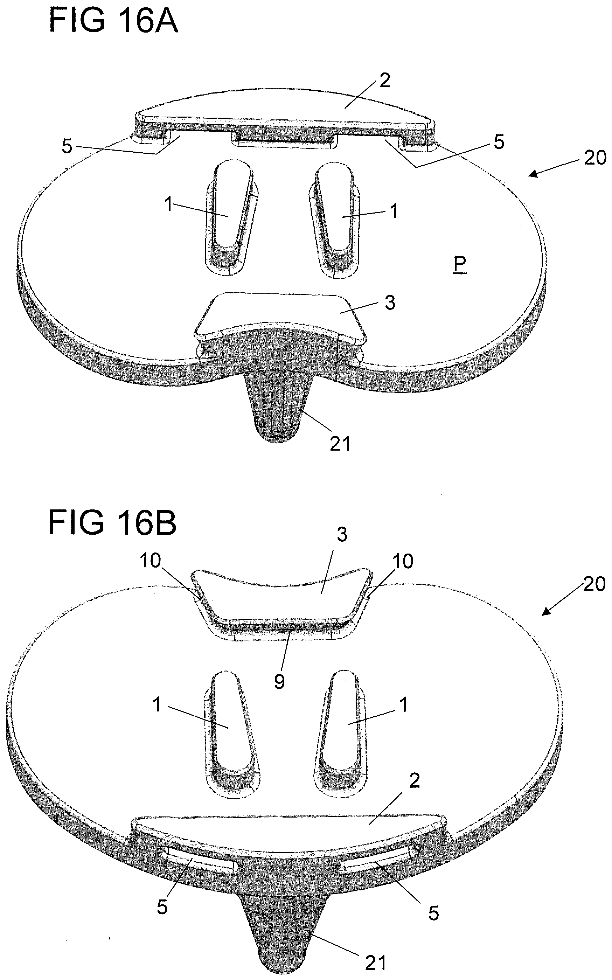

In other embodiments, as e.g. shown in Fig. 9, the central fixation elements 1 had a shape with two symmetry axes, one along the long axis and on axis perpendicular to this axis. In Fig 16A, 16B an embodiment of a tibial tray 20 is shown which deviates from this pattern.

In Fig. 16A, 16B an embodiment is shown in which the central fixation elements 1 have a wider lateral cross-section towards the anterior side than towards the posterior side. Hence, the long sidewalls of each of the central fixation elements 1 are converging towards the anterior side. The inner sidewalls of the central fixation elements 1 are not parallel and are diverging towards the posterior of the tibial tray 20, whereas the outer sidewalls are essentially parallel.

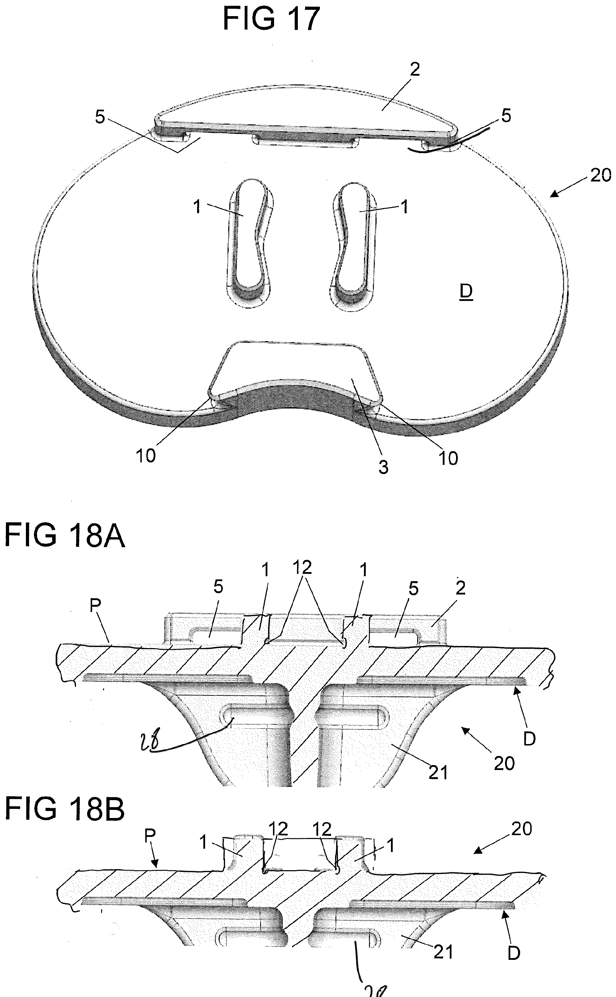

In Fig. 17, a variant embodiment of the tibial tray 20 is shown, as the central fixation elements 1 are shaped differently. Here, the outer sidewalls of the central fixation elements 1 are parallel to each other and there are two symmetry axes, as in the embodiment shown in Fig. 9. In the embodiment of Fig. 17, the inner sidewalls are not linear over the complete lengths. Towards the anterior and posterior side, the sidewalls are converging, towards the middle there are diverging.

The anterior fixation element 2 of the embodiments shown in Fig. 16A, 16B and 17 are shaped as the embodiment shown in Fig. 9. But the posterior fixation elements 3 of the embodiments shown in Fig. 16A, 16B and 17 are shaped differently.

The sidewalls of the posterior element 3 are tilted by an angle between 0 and 45°, in particular between 0 and 30° relative to a plane that is perpendicular to the proximal plane P. In addition or alternatively, the sidewalls and the anterior wall of the of the at least one posterior element 3 comprise an undercut.

In Fig. 18A, 18B, cross-sections in a frontal plane N (see e.g. Fig. 9A) are shown for one embodiment. Fig. 18A shows a view in the anterior direction, i.e. the anterior fixation element 2 can be seen behind the central fixation elements 1, which are shown in cross-section. Fig. 18B shows a view onto the same frontal plane N but into the posterior direction.

The cross-sections of the central fixation elements 1 show that they comprise central fixation element recesses 12 (undercuts) at the transition to the proximal surface P and along the inner sidewalls. The central fixation element recesses 12 here grooves with a constant radius. In a different embodiment not shown here, the central fixation element recesses 12 can be also on the outer sidewalls of the central fixation elements 1 or only in the outer sidewalls.

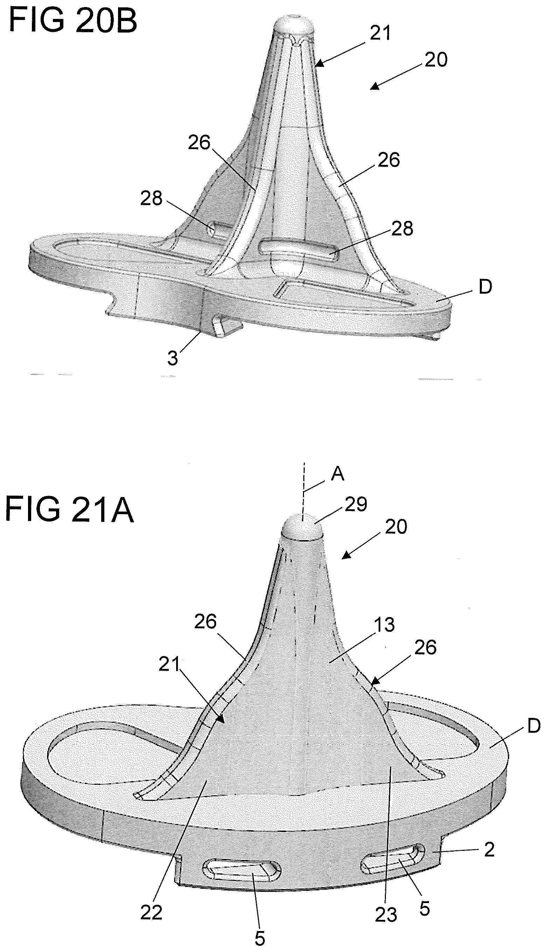

Fig. 18A, 18B also show cement holding structures 28 which will be described in more detail in connection with Fig. 20A and 20B.

Fig. 19 shows a schematic representation of a view into the anterior direction with the tibial tray structure 21 (here a T-structure) pointing upwards in the distal direction. This representation is not shown as a particular embodiment of a tibial tray 20 but it is intended to indicate how the shape of the outer rim 26 of the sidewalls (e.g. as in Fig. 4 and 5) can be generated. In this exemplary embodiment the outer rim 26 follows a strictly monotonous curve with inflection points. A possible generation of this curve is described below by a using a number of line segments M1, M2, M3, M4, M5 which are connecting the distal plane D with the distal tip of the tibial tray shaft structure 21.

The first line segment M1 is connecting the distal plane D with a first point P1 in the distal plane D. The first point P1 is laterally positioned at about 68% of the distance from the medial plane M (see Fig. 9A) and the and the outermost point of the lateral rim. The inclination of the first line segment M1 is about 20° as measured from the distal plane D. In alternative embodiments, the first point P1 is laterally positioned within a range of 55 to 75% of the distance from the medial plane M to the outermost point of the lateral rim. In an alternative embodiment, the inclination of the first line segment M1 can also be in the range between 15° to 30° as measured from the distal plane D. The embodiments can be combined.

The second line segment M2 is connecting the first point P1 with a second point P2 above the distal plane D. The second point P2 is vertically positioned at about 15% of the distance from the distal plane D to the parallel plane with the distal-most point of the tibial tray shaft structure 21. The inclination of the second line segment M2 is about 40° as measured from the distal plane D. In alternative embodiments, the second point P2 is vertically positioned within a range of 10 to 20% of the distance from the distal plane D to the parallel plane with the distal-most point of the tibial tray shaft structure 21. In an alternative embodiment, the inclination of the second line segment M2 can also be in the range between 30° to 50° as measured from the distal plane D. The embodiments can be combined.

The third line segment M3 is connecting the second point P2 with a third point P3 above the distal plane D. The third point P3 is vertically positioned at about 33% of the distance from the distal plane D to the parallel plane with the distal-most point of the tibial tray shaft structure 21. The inclination of the third line segment M3 is about 60° as measured from the distal plane D. In alternative embodiments, the third point P3 is vertically positioned within a range of 25 to 40% of the distance from the distal plane D to the parallel plane with the distal-most point of the tibial tray shaft structure 21. In an alternative embodiment, the inclination of the third line segment M3 can also be in the range between 45° to 70° as measured from the distal plane D. The embodiments can be combined.

The fourth line segment M4 is connecting the third point P3 with a fourth point P4 above the distal plane D. The fourth point P4 is vertically positioned at about 50% of the distance from distal plane D to the parallel plane with the distal-most point of the tibial tray shaft structure 21. The inclination of the fourth line segment M4 is about 50° as measured from the distal plane D. In alternative embodiments, the fourth point P4 is vertically positioned within a range of 40 to 60% of the distance from the distal plane D to the parallel plane with the distal-most point of the tibial tray shaft structure 21. In an alternative embodiment, the inclination of the fourth line segment M4 can also be in the range between 40° to 60° as measured from the distal plane D. The embodiments can be combined.

The fifth line segment M5 is connecting the fourth point P4 with a point at the central structure of the tibial tray shaft 21. The inclination of the fifth line segment M5 is about 75° as measured from the distal plane D. In other embodiments, the inclination of the fifth line segment M5 is in the range between 50 to 80° as measured from the distal plane D. The embodiments can be combined.

The line segments M1, M2, M3, M4, M5 have varying inclinations, so that in roughly the middle, the inclination of the fourth line segment M4 is steeper than that of the third line segment M3.

The points defining the line segments can be fitted with smooth curve, such as a polynom or a spline curve to generate the smooth outline of the outer rim 26 of one of the tibial tray shaft structures 21, i.e. the two lateral shaft all elements in the T-structure are shown in Fig. 19.

Fig. 20A and 20B show an embodiment of a tibial tray 20 in perspective views. Essentially, the embodiment has the features as described above, but the tibial shaft structure 21 - here a T-structure - comprises relatively close to the distal surface D cement holding structures 23. The anterior side - best seen in Fig. 20A - comprises a groove as cement holding structure 28 across the two wall elements. The posterior side - best seen in Fig. 20B - comprises two grooves as cement holding structure 28 each extending over two wall elements, i.e. traversing the rounded corner between the wall elements.

The cement holding structures 28 are designed here as rounded grooves which are easy to manufacture. In other embodiments, they can also comprise or consist of other shapes, e.g. grooves with sharp corners or holes with round or polygonal cross-sections.

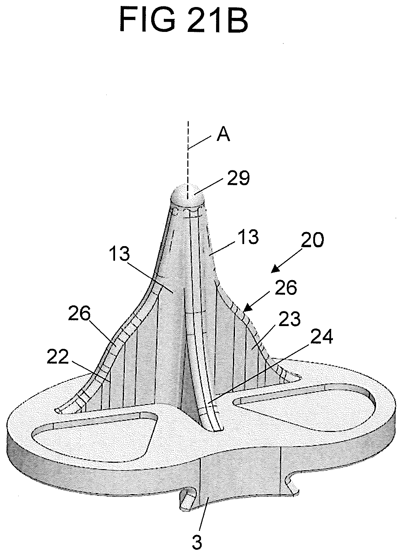

In Fig. 21A, 21B, an embodiment of a tibial tray 20 is shown which is a variant of the above-described embodiments, such as the ones shown in Fig. 20A, 20B, but here without the cement holding structure 23. The tibial tray shaft structure 21 comprises a vertical cylinder-shaped base shaft structure 29 extending around the axis A of the tibial tray shaft structure 21. The diameter of this base shaft structure 29 is between 5 to 8 mm, in particular depending on the size of the tibial tray 20. The diameter can vary in discrete steps, such as 5 mm, 6 mm, 7mm, and 8 mm. The cross-section of the base shaft structure 29 does not have to be circular. Other shapes, as e.g. elliptical or polygonal, are also possible.

As discussed above, three wall elements 22, 23, 24 (Y-structure, T-structure) or four wall elements 22, 23, 24, 25 (X-structure) extend radially away from that base shaft structure 29. The angles between the wall elements 22, 23, 24, 25 are e.g. described in connection with Fig. 15A, 15B, 15C. The angular spaces between the wall elements 22, 23, 24, 25 are smoothly filled with curved surfaces 13, e.g. connecting adjacent wall elements 22, 23, 24, 25. Fig. 21B shows the posterior view of a tibial tray 20 (T-structure). The radius of the curved surface 13 close to the base shaft structure 29 is between 1 and 9 mm, in particular between 3 and 5 mm. Similar curved surfaces with the same radii would be used between wall elements in X-structures or Y-structures.

Fig. 21A shows the anterior view of the T-structure shown in Fig. 21B. Here, the cylindrical base shaft structure 29 extends into the anterior direction beyond the two frontal wall elements 22, 23. The curved surface 23 at the frontal side here is between 10 mm and 30 mm, in particular 20 mm.

Those curved surfaces 13 prevent sharp corners which improves inter alia the insertabilty of the tibial tray.

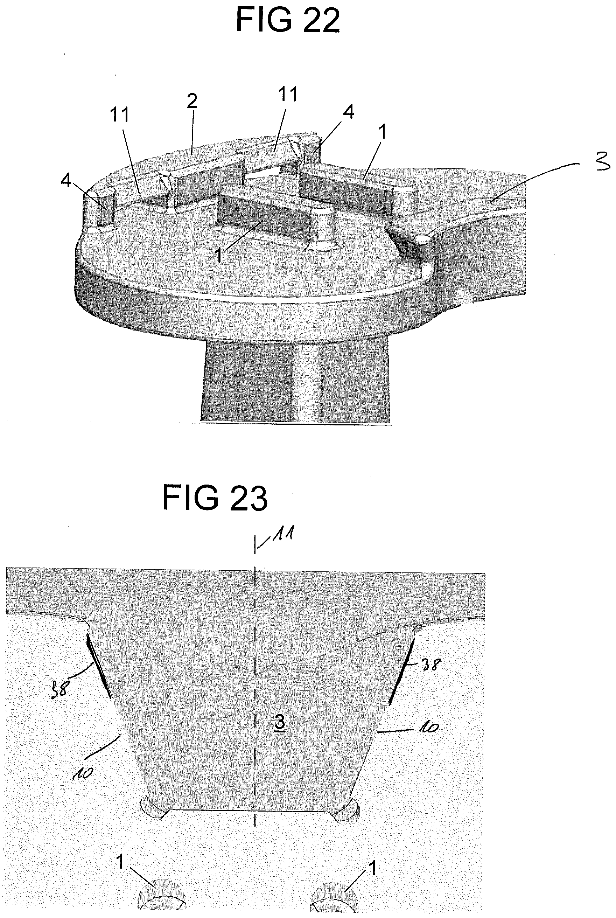

In Fig. 22, the proximal surface P of a tibial tray 20 is shown which comprises a variation in the design of the anterior fixation element 2 over the embodiments described above. The central fixation elements 1 and the posterior fixation element 3 have been described in one way or the other e.g. in Fig. 9 or 16A, so that reference can be made to the above description.

The anterior fixation element 2 comprises two form-fitting elements 5 for receiving a corresponding part of the tibial insert 30 (not shown here, see Fig. 12A, 12B e.g. for the snap-in process of the tibial tray). The form-fitting elements 5 are essentially holes with a rectangular cross-section.

As the tibial insert 30 is inserted from the top using its rear part and the posterior fixation device 3 as fulcrum, the form-fitting elements 31 for the anterior fixation have a shape which corresponds to the form-fitting elements 5 (e.g. the rectangular holes).

To ease the snapping in of the tibial insert 30 (not shown here), guiding surfaces 11, are provided for on the proximal surface of the anterior fixation device 5. The guiding surfaces 11 here are planes inclined by 5° to 45° against the horizontal plane, in particular inclined by 30°. In other embodiments, not shown here. The guiding surface are curved or have a plane part.

The form-fitting elements 31 of the tibial insert 30 protrude into the anterior direction. When the tibial insert 30 is about to be snapped-in, the form-fitting elements 31 first rest on the upper parts of the guiding surfaces 11. When pressure in the distal direction is applied in the tibial insert 30, the form-fitting elements 31 are guided downwards along the guiding surfaces 11 and eventually snap into the holes forming the form-fitting elements of the anterior fixation element 5 for a secure fit. The guiding surfaces 11 prevent compromising the form-fitting elements 5 during insertion and make a secure snap-in easier.

The lateral width of the guiding surface is the same or about the same as the lateral width of the form-fitting elements 5 in the anterior fixation element 2.

In Fig. 23, a detail of the posterior fixation element 3 is shown in a top view, i.e. in the direction of the proximal surface P.

The cross-section of the posterior fixation element 3 comprises two sidewalls 10 that are inclined towards the medial plane M. Each of the sidewalls 10 is angled by about 20° against the medial plane M. In the embodiment shown, there is a deliberate angular mismatch between the sidewalls 10 of the posterior fixation element 3 and the corresponding sidewalls of the tibial insert 30 (not shown here).

If, for example the sidewalls of the tibial insert 30, which are intended to get into contact or into a form-fit with the sidewalls 10 of the posterior fixation element 3, have a lightly different inclination than the sidewalls 10, an angular mismatch is created causing a closer contact or a force-fit at some parts along the sidewalls 10 and a looser fit in others.

In the embodiment shown in Fig. 23, the angle of the sidewalls of posterior fixation element 3 is wider (e.g. by about 1°) than the corresponding angle in the recess in the tibial insert 30. This causes a form-fit, i.e. a tighter fit in the posterior area. The press-fitting region 38 in this area is indicated in Fig. 23. Not only enables this angular mismatch an easier assembly, but it also reduces micromovements in the posterior regions.

In other embodiments the press-fit region 38 is created by widening the posterior part of the posterior fixation element 3.

### Reference signs

- 1: central fixation element
- 2: anterior fixation element
- 3: posterior fixation element
- 4: posterior wall of anterior fixation element
- 5: form-fitting element of anterior fixation element
- 6: undercut in wall of posterior fixation element
- 7: posterior wall of posterior fixation element
- 8: posterior rim of tibial tray
- 9: anterior wall of posterior fixation element
- 10: sidewall of posterior fixation element
- 11: guiding surface for a tibial insert on the anterior fixation element
- 12: central fixation element recess
- 13: curved surface between shaft wall elements
- 14: recess in distal surface

- 20: tibial tray
- 21: tibial tray shaft structure
- 22: first shaft wall element of tibial tray shaft structure
- 23: second shaft wall element of tibial tray shaft structure
- 24: third shaft wall element of tibial tray shaft structure
- 25: fourth shaft wall element of tibial tray shaft structure
- 26: outer rim of shaft wall element
- 27: inflection point on outer rim of shaft wall element
- 28: cement holding structure, recess structure in shaft wall element for bone cement
- 29: base shaft structure

- 30: tibial insert
- 31: form-fitting element for anterior fixation element
- 32: recess for posterior fixation element
- 33: relief notch
- 34: notch /groove in tibial insert for linear central fixation element
- 35: anterior wall of recess
- 36: sidewall of recess
- 37: press-fit surface
- 38: press-fit region due to angular mismatch
- 40: femoral part

- 100: artificial knee joint

- A: axis of the tibial tray shaft structure
- C: cross-section plane
- D: distal surface of tibial tray
- DD: distal direction
- D1: distance
- H: height of a fixation element
- L: height of tibial tray structure measured from distal surface
- L1: lateral point of the proximal surface
- L2: lateral point of the proximal surface
- M: medial plane of proximal surface of tibial tray
- M1: first line segment defining the outer rim of shaft element
- M2: second line segment defining the outer rim of shaft element
- M3: third line segment defining the outer rim of shaft element
- M4: fourth line segment defining the outer rim of shaft element
- M5: fifth line segment defining the outer rim of shaft element
- N: frontal plane perpendicular to the medial plane
- P: proximal surface of tibial tray
- P1: first point between line segments defining the outer rim of shaft element
- P2: second point between line segments defining the outer rim of shaft element
- P3: third point between line segments defining the outer rim of shaft element
- P4: fourth point between line segments defining the outer rim of shaft element
- R: rim of distal surface
- R1: radius,
- R2: radius
- Rant: anterior radius
- Rlat: laterial radius
- Rpost: posterior radius
- S: smooth surface
- T: thickness of shaft wall element

- α: angle between sidewalls and the anterior wall of the anterior fixation element in a plane parallel to the proximal surface
- β: angle of undercut
- γ₁: angle between first and second wall structure of a tibial tray structure
- γ₂: angle between third and fourth all structure of a tibial tray structure

## Claims

1. Tibial tray (20) with a distal surface (D), wherein a tibial tray shaft structure (21) extends away from the distal surface (D) in the distal direction (DD) by a height (L), wherein
the tibial tray structure (21) comprises at least three shaft wall elements (22, 23, 24, 25) which are so arranged relative to each other that the cross-sections of the at least three shaft wall elements (22, 23, 24, 25) in a plane perpendicular to the distal direction (DD) are T-shaped, Y-shaped or X-shaped, and
the area of the cross-sections decreases strictly monotonously from the distal surface (D) in the distal direction (DD), wherein the at least three shaft wall elements each comprise an outer rim that is a thin face of the respective shaft wall element, wherein said outer rim has a curvature,
**characterised in that,**
any change of a tangent angle (γ) at said outer rim (26) of the at least three shaft wall elements (22, 23, 24, 25) is less than 30° over a change in distance in the distal direction of 10% of the height (L) of the tibial tray structure (21), in particular any tangent angle (γ) of the outer rim (26) of the at least three shaft wall elements (22, 23, 24, 25) within a third of the height (L) of the tibial tray structure (21) measured from the distal surface (D).

2. Tibial tray (20) according to claim 1, wherein the outer rims (26) of the at least three shaft wall elements (22, 23, 24, 25) converge strictly monotonously in the distal direction (DD) and towards an axis (A) of the tibial tray structure (21) and/or have at least one inflection point (27), in particular three inflection points (27).

3. Tibial tray (20) according to at least one of the preceding claims, wherein at least 50% of the volume of the tibial tray structure (21) is located within a third of the height (L) of the tibial tray structure (21) measured from the distal surface (D).

4. Tibial tray (20) according to at least one of the preceding claims, wherein at least one of the shaft wall elements (22, 23, 24, 25) has wall thickness (T) between 1 and 6 mm, in particular between 2 and 5 mm, in particular for ceramic materials between 1 and 5 mm, measured at the point farthest way from the axis (A) and on the distal surface (D).

5. Tibial tray (20) according to at least one of the preceding claims, wherein the radius (γ) at the transition from the outer rim (26) of at least one of the at least three shaft wall elements (22, 23, 24, 25) to the sidewalls of the at least one of the at least three shaft wall element is in the range of 0,5 to 4 mm.

6. Tibial tray (20) according to at least one of the preceding claims, wherein at least one of the at least three shaft wall elements (22, 23, 24, 25) comprises at least cement holding structure, in particular at least one recess structure (28) for retaining bone cement, in particular on the anterior and / or posterior side.

7. Tibial tray (20) according to at least one of the preceding claims, wherein the transition point from the outer rim (26) into the tibial shaft structure (21) in the distal surface (D) is located at least 5 mm from the lateral sides and / or the posterior side of the distal surface (D).

8. Tibial tray (20) according to at least one of the preceding claims, wherein the tibial tray structure (21) comprises a surface (S) extending between at least two of the outer rims (26) of the at least three shaft wall elements (21) or between two side walls of the at least three shaft wall elements (21).

9. Tibial tray (20) according to at least one of the preceding claims, wherein at least one of
the radius (R1) at the transition from the distal surface (D) to at least one of the at least three shaft wall elements (22, 23, 24, 25) and / or the smooth surface (S) is in the range of 1 to 4 mm,
the axis (A) of the tibial tray shaft structure (21) is located on the distal surface (D) at one third of the distance between the anterior rim to the posterior rim, measured from the anterior rim, and
the angle (γ₁) between the shaft elements (22, 23) is between 30° to 120° on the posterior side and the angle (γ₂) between the shaft wall elements (24, 25) is between 90° and 180° on the anterior side.

10. Tibial tray (20) according to at least one of the preceding claims, wherein the tibial tray shaft structure (21) comprises at least one cement holding structure (28), in particular a hole, a groove and / or a recess without any undercut, optionally wherein the at least one cement holding structure (28) is located within the vertical two thirds of the tibial tray shaft structure (21) measured from the distal surface (D) in the distal direction (DD), in particular within the first third.

11. Tibial tray (20) according to at least one of the preceding claims, wherein at least one anterior fixation element (2), in particular exactly one anterior fixation element (2), is positioned at the anterior side of the proximal surface (P), the posterior side of the at least one anterior fixation element (2) comprising a wall (4) which in a plane parallel to the proximal surface (P) is perpendicular to the medial plane (M) of the proximal surface (P),
optionally wherein the at least one anterior fixation element (2) comprises at least one form-fitting element (5), in particular at least one cavity in the posterior wall (4) of the at least one anterior fixation element (2) and / or at least one channel through the at least one anterior fixation element (2), for a corresponding form-fitting element (31) of the tibial insert (30), the at least one form-fitting element (5) of the at least one anterior fixation element (2) being in particular positioned in a region in which the load transfer is at less than 70%, in particular 50%, from the maximum load transfer,
optionally wherein the at least one anterior fixation element (2) comprises at least on guiding surface (11) for a tibial insert (30), in particular the guiding surface (11) having a curved and / or a plane part, in a proximal direction of the at least form-fitting element (5),
optionally wherein the at least one guiding surface (11) comprises a plane part or is a plane which is inclined relative to the horizontal plane by an angle in the range between 5 to 45°, in particular 30°.

12. Tibial tray (20) according to at least one of the preceding claims, with a proximal surface (P), the proximal surface (P) comprising at least two, in particular exactly two central fixation elements (1) for enabling a connection with the tibial insert (30) as a further part of an artificial knee joint, wherein the at least two central fixation elements (1) are protrusions from the proximal surface (P) in the proximal direction and the at least two central fixation elements (1) are positioned symmetrically and / or parallel to a medial plane (M) of the proximal surface (P), the medial plane (M) being perpendicular to the proximal surface (P) and intersecting the proximal surface (P) in the middle between two most lateral points (L1, L2) of the proximal surface (P).

13. Tibial tray (20) according to at least one of the preceding claims, wherein the at least two central fixation elements (1) comprise a central fixation element recess (12).

14. Tibial tray (20) according to at least one of the preceding claims, wherein at least one, in particular exactly one posterior fixation element (3) is positioned symmetrically to the medial plane (M) of the proximal surface (P) comprising a posterior wall (7) flush with the posterior rim (8) of the tibial tray (20) and / or comprising an anterior wall (9) which is essentially positioned parallel to the posterior wall (4) of the at least one anterior fixation element (2),
optionally wherein the sidewalls (10) and / or the anterior wall (9) of the at least one posterior element (3) are tilted by an angle between 0° and 45°, in particular between 0 and 30°, more particular by an angle of 10°, relative to a plane which is perpendicular to the proximal plane (P) and / or the sidewalls (10) and the anterior wall (9) of the at least one posterior element (3) comprises at an undercut.

15. Tibial tray (20) according to at least one of the preceding claims, wherein the at least two central fixation elements (1) are oriented symmetrically and / or parallel to the medial plane (M) or the at least two fixation elements (1) are oriented asymmetrically relative to the medial plane (M).

16. Tibial tray (20) according to at least one of the preceding claims, wherein it is manufactured completely or in part from ceramic, a polymer material or a metal.

17. Artificial knee joint (100) comprising a tibial tray (20) according to at least one of the claims 1 to 16 and a tibial insert (30), optionally wherein there is at least one press-fitting region (38) between the posterior fixation element (3) and the tibial insert (20), in particular created by an angular mismatch between two walls.

## Patentansprüche

1. Tibia-Basisplatte (20) mit einer distalen Oberfläche (D), wobei sich eine Tibia-Basisplatten-Schaftstruktur (21) von der distalen Oberfläche (D) in distaler Richtung (DD) um eine Höhe (L) weg erstreckt, wobei
die Tibia-Basisplatten-Schaftstruktur (21) zumindest drei Schaftwandelemente (22, 23, 24, 25) umfasst, die relativ zueinander so angeordnet sind, dass die Querschnitte der zumindest drei Schaftwandelemente (22, 23, 24, 25) in einer Ebene senkrecht zur distalen Richtung (DD) T-förmig, Y-förmig oder X-förmig sind,
die Fläche der Querschnitte von der distalen Oberfläche (D) in distaler Richtung (DD) streng monoton abnimmt, wobei die zumindest drei Schaftwandelemente jeweils einen Außenrand umfassen, der eine schmale Fläche des jeweiligen Schaftwandelements ist, wobei der Außenrand eine Krümmung aufweist, **dadurch gekennzeichnet, dass** jede Änderung eines Tangentenwinkels (γ) an dem Außenrand (26) der zumindest drei Schaftwandelemente (22, 23, 24, 25) weniger als 30° über einer Änderung des Abstands in der distalen Richtung von 10 % der Höhe (L) der Tibia-Basisplatten-Schaftstruktur (21) beträgt, insbesondere jeder Tangentenwinkel (γ) des Außenrands (26) der zumindest drei Schaftwandelemente (22, 23, 24, 25) innerhalb eines Drittels der Höhe (L) der Tibia-Basisplatten-Schaftstruktur (21), gemessen von der distalen Oberfläche (D).

2. Tibia-Basisplatte (20) nach Anspruch 1, wobei die Außenränder (26) der zumindest drei Schaftwandelemente (22, 23, 24, 25) in distaler Richtung (DD) und hin zu einer Achse (A) der Tibia-Basisplatten-Schaftstruktur (21) streng monoton konvergieren und/oder zumindest einen Wendepunkt (27), insbesondere drei Wendepunkte (27), aufweisen.

3. Tibia-Basisplatte (20) nach zumindest einem der vorstehenden Ansprüche, wobei zumindest 50 % des Volumens der Tibia-Basisplatten-Schaftstruktur (21) innerhalb eines Drittels der Höhe (L) der Tibia-Basisplatten-Schaftstruktur (21), gemessen von der distalen Oberfläche (D), angeordnet sind.

4. Tibia-Basisplatte (20) nach zumindest einem der vorstehenden Ansprüche, wobei zumindest eines der Schaftwandelemente (22, 23, 24, 25) eine Wanddicke (T) zwischen 1 und 6 mm, insbesondere zwischen 2 und 5 mm, insbesondere für keramische Materialien zwischen 1 und 5 mm, aufweist, gemessen an dem am weitesten von der Achse (A) entfernten Punkt und an der distalen Oberfläche (D).

5. Tibia-Basisplatte (20) nach zumindest einem der vorstehenden Ansprüche, wobei der Radius (γ) am Übergang von dem Außenrand (26) zumindest eines der zumindest drei Schaftwandelemente (22, 23, 24, 25) zu den Seitenwänden des zumindest einen der zumindest drei Schaftwandelemente im Bereich von 0,5 bis 4 mm liegt.

6. Tibia-Basisplatte (20) nach zumindest einem der vorstehenden Ansprüche, wobei zumindest eines der zumindest drei Schaftwandelemente (22, 23, 24, 25) zumindest eine Zementhaltestruktur umfasst, insbesondere zumindest eine Aussparungsstruktur (28) zum Aufnehmen von Knochenzement, insbesondere auf der anterioren und/oder posterioren Seite.

7. Tibia-Basisplatte (20) nach zumindest einem der vorstehenden Ansprüche, wobei der Übergangspunkt von dem Außenrand (26) in die Tibia-Schaftstruktur (21) in der distalen Oberfläche (D) zumindest 5 mm von den lateralen Seiten und/oder der posterioren Seite der distalen Oberfläche (D) entfernt angeordnet ist.

8. Tibia-Basisplatte (20) nach zumindest einem der vorstehenden Ansprüche, wobei die Tibia-Basisplatten-Schaftstruktur (21) eine Oberfläche (S) umfasst, die sich zwischen zumindest zwei der Außenränder (26) der zumindest drei Schaftwandelemente (21) oder zwischen zwei Seitenwänden der zumindest drei Schaftwandelemente (21) erstreckt.

9. Tibia-Basisplatte (20) nach zumindest einem der vorstehenden Ansprüche, wobei
der Radius (R1) am Übergang von der distalen Oberfläche (D) zu zumindest einem der zumindest drei Schaftwandelemente (22, 23, 24, 25) und/oder der glatten Oberfläche (S) im Bereich von 1 bis 4 mm liegt, und/oder
die Achse (A) der Tibia-Basisplatten-Schaftstruktur (21) an der distalen Oberfläche (D) bei einem Drittel des Abstands zwischen dem anterioren Rand und dem posterioren Rand, gemessen von dem anterioren Rand, angeordnet ist, und/oder
der Winkel (γ₁) zwischen den Schaftelementen (22, 23) auf der posterioren Seite zwischen 30° und 120° beträgt und der Winkel (γ₂) zwischen den Schaftwandelementen (24, 25) auf der anterioren Seite zwischen 90° und 180° beträgt.

10. Tibia-Basisplatte (20) nach zumindest einem der vorstehenden Ansprüche, wobei die Tibia-Basisplatten-Schaftstruktur (21) zumindest eine Zementhaltestruktur (28), insbesondere ein Loch, eine Nut und/oder eine Aussparung ohne jegliche Hinterschneidung, umfasst, wobei optional die zumindest eine Zementhaltestruktur (20) innerhalb der vertikalen zwei Drittel der Tibia-Basisplatten-Schaftstruktur (21), gemessen von der distalen Oberfläche (D) in distaler Richtung (DD), insbesondere innerhalb des ersten Drittels, angeordnet ist.

11. Tibia-Basisplatte (20) nach zumindest einem der vorstehenden Ansprüche, wobei zumindest ein anteriores Fixierelement (2), insbesondere genau ein anteriores Fixierelement (2), an der anterioren Seite der proximalen Oberfläche (P) positioniert ist, wobei die posteriore Seite des zumindest einen anterioren Fixierelements (2) eine Wand (4) umfasst, die in einer Ebene parallel zu der proximalen Oberfläche (P) senkrecht zu der medialen Ebene (M) der proximalen Oberfläche (P) ist,
wobei optional das zumindest eine anteriore Fixierelement (2) zumindest ein Formschlusselement (5), insbesondere zumindest einen Hohlraum in der posterioren Wand (4) des zumindest einen anterioren Fixierelements (2) und/oder zumindest einen Kanal durch das zumindest eine anteriore Fixierelement (2), für ein entsprechendes Formschlusselement (31) des Tibia-Einsatzes (30) umfasst, wobei das zumindest eine Formschlusselement (5) des zumindest einen anterioren Fixierelements (2) insbesondere in einem Bereich positioniert ist, in dem die Lastübertragung weniger als 70 %, insbesondere 50 %, der maximalen Lastübertragung beträgt,
wobei optional das zumindest eine anteriore Fixierelement (2) zumindest eine Führungsfläche (11) für einen Tibia-Einsatz (30) umfasst, wobei insbesondere die Führungsfläche (11) einen gekrümmten und/oder einen ebenen Teil in einer proximalen Richtung des zumindest einen Formschlusselements (5) aufweist,
wobei optional die zumindest eine Führungsfläche (11) einen ebenen Teil umfasst oder eine Ebene ist, die relativ zur horizontalen Ebene um einen Winkel im Bereich zwischen 5 und 45°, insbesondere 30°, geneigt ist.

12. Tibia-Basisplatte (20) nach zumindest einem der vorstehenden Ansprüche, mit einer proximalen Oberfläche (P), wobei die proximale Oberfläche (P) zumindest zwei, insbesondere genau zwei zentrale Fixierelemente (1) zum Ermöglichen einer Verbindung mit dem Tibia-Einsatz (30) als einem weiteren Teil eines künstlichen Kniegelenks umfasst, wobei die zumindest zwei zentralen Fixierelemente (1) Vorsprünge von der proximalen Oberfläche (P) in proximaler Richtung sind und die zumindest zwei zentralen Fixierelemente (1) symmetrisch und/oder parallel zu einer medialen Ebene (M) der proximalen Oberfläche (P) positioniert sind, wobei die mediale Ebene (M) senkrecht zur proximalen Oberfläche (P) ist und die proximale Oberfläche (P) in der Mitte zwischen zwei am weitesten lateral gelegenen Punkten (L1, L2) der proximalen Oberfläche (P) schneidet.

13. Tibia-Basisplatte (20) nach zumindest einem der vorstehenden Ansprüche, wobei die zumindest zwei zentralen Fixierelemente (1) eine zentrale Fixierelement-Aussparung (12) umfassen.

14. Tibia-Basisplatte (20) nach zumindest einem der vorstehenden Ansprüche, wobei zumindest ein, insbesondere genau ein posteriores Fixierelement (3) symmetrisch zu der medialen Ebene (M) der proximalen Oberfläche (P) positioniert ist, umfassend eine posteriore Wand (7), die bündig mit dem posterioren Rand (8) der Tibia-Basisplatte (20) ist, und/oder umfassend eine anteriore Wand (9), die im Wesentlichen parallel zu der posterioren Wand (4) des zumindest einen anterioren Fixierelements (2) positioniert ist,
wobei optional die Seitenwände (10) und/oder die anteriore Wand (9) des zumindest einen posterioren Elements (3) um einen Winkel zwischen 0° und 45°, insbesondere zwischen 0 und 30°, besonders insbesondere um einen Winkel von 10°, relativ zu einer Ebene geneigt sind, die senkrecht zur proximalen Ebene (P) ist, und/oder die Seitenwände (10) und die anteriore Wand (9) des zumindest einen posterioren Elements (3) eine Hinterschneidung umfassen.

15. Tibia-Basisplatte (20) nach zumindest einem der vorstehenden Ansprüche, wobei die zumindest zwei zentralen Fixierelemente (1) symmetrisch und/oder parallel zu der medialen Ebene (M) ausgerichtet sind oder die zumindest zwei Fixierelemente (1) asymmetrisch relativ zur medialen Ebene (M) ausgerichtet sind.

16. Tibia-Basisplatte (20) nach zumindest einem der vorstehenden Ansprüche, wobei es vollständig oder teilweise aus Keramik, einem Polymermaterial oder einem Metall hergestellt ist.

17. Künstliches Kniegelenk (100), umfassend ein Tibia-Basisplatte (20) nach zumindest einem der Ansprüche 1 bis 16 und einen Tibia-Einsatz (30), wobei optional zumindest ein Presspassungsbereich (38) zwischen dem posterioren Fixierelement (3) und dem Tibia-Einsatz (20) vorhanden ist, insbesondere erzeugt durch eine Winkelabweichung zwischen zwei Wänden.

## Revendications

1. Plateau tibial (20) avec une surface distale (D), dans lequel une structure de tige du plateau tibial (21) s'étend à l'écart de la surface distale (D) dans la direction distale (DD) sur une hauteur (L), dans lequel
la structure de tige du plateau tibial (21) comprend au moins trois éléments de paroi de tige (22, 23, 24, 25) qui sont agencés les uns par rapport aux autres de telle sorte que les sections transversales des au moins trois éléments de paroi de tige (22, 23, 24, 25), dans un plan perpendiculaire à la direction distale (DD), sont en forme de T, en forme de Y ou en forme de X,
l'aire des sections transversales diminue de façon strictement monotone depuis la surface distale (D) dans la direction distale (DD), dans lequel les au moins trois éléments de paroi de tige comprennent chacun un bord extérieur qui est une face mince de l'élément de paroi de tige respectif, dans lequel ledit bord extérieur présente une courbure, **caractérisé en ce que** toute variation d'un angle tangent (γ) audit bord extérieur (26) des au moins trois éléments de paroi de tige (22, 23, 24, 25) est inférieure à 30° sur une variation de distance dans la direction distale de 10 % de la hauteur (L) de la structure de tige du plateau tibial (21), en particulier tout angle tangent (γ) du bord extérieur (26) des au moins trois éléments de paroi de tige (22, 23, 24, 25) à l'intérieur d'un tiers de la hauteur (L) de la structure de tige du plateau tibial (21), mesurée depuis la surface distale (D).

2. Plateau tibial (20) selon la revendication 1, dans lequel les bords extérieurs (26) des au moins trois éléments de paroi de tige (22, 23, 24, 25) convergent de façon strictement monotone dans la direction distale (DD) et vers un axe (A) de la structure de tige du plateau tibial (21) et/ou présentent au moins un point d'inflexion (27), en particulier trois points d'inflexion (27).

3. Plateau tibial (20) selon au moins l'une des revendications précédentes, dans lequel au moins 50 % du volume de la structure de tige du plateau tibial (21) est situé à l'intérieur d'un tiers de la hauteur (L) de la structure de tige du plateau tibial (21), mesurée depuis la surface distale (D).

4. Plateau tibial (20) selon au moins l'une des revendications précédentes, dans lequel au moins l'un des éléments de paroi de tige (22, 23, 24, 25) présente une épaisseur de paroi (T) comprise entre 1 et 6 mm, en particulier entre 2 et 5 mm, en particulier pour des matériaux céramiques entre 1 et 5 mm, mesurée au point le plus éloigné de l'axe (A) et sur la surface distale (D).

5. Plateau tibial (20) selon au moins l'une des revendications précédentes, dans lequel le rayon (γ) à la transition entre le bord extérieur (26) d'au moins l'un des au moins trois éléments de paroi de tige (22, 23, 24, 25) et les parois latérales de l'au moins un des au moins trois éléments de paroi de tige est compris dans la plage de 0,5 à 4 mm.

6. Plateau tibial (20) selon au moins l'une des revendications précédentes, dans lequel au moins l'un des au moins trois éléments de paroi de tige (22, 23, 24, 25) comprend au moins une structure de retenue de ciment, en particulier au moins une structure d'évidement (28) pour retenir du ciment osseux, en particulier sur le côté antérieur et/ou postérieur.

7. Plateau tibial (20) selon au moins l'une des revendications précédentes, dans lequel le point de transition du bord extérieur (26) vers la structure de tige du plateau tibial (21) dans la surface distale (D) est situé à au moins 5 mm des côtés latéraux et/ou du côté postérieur de la surface distale (D).

8. Plateau tibial (20) selon au moins l'une des revendications précédentes, dans lequel la structure de tige du plateau tibial (21) comprend une surface (S) s'étendant entre au moins deux des bords extérieurs (26) des au moins trois éléments de paroi de tige (21) ou entre deux parois latérales des au moins trois éléments de paroi de tige (21).

9. Plateau tibial (20) selon au moins l'une des revendications précédentes, dans lequel au moins l'un parmi
le rayon (R1) à la transition entre la surface distale (D) et au moins l'un des au moins trois éléments de paroi de tige (22, 23, 24, 25) et/ou la surface lisse (S) est compris dans la plage de 1 à 4 mm,
l'axe (A) de la structure de tige du plateau tibial (21) est situé sur la surface distale (D) à un tiers de la distance entre le bord antérieur et le bord postérieur, mesurée depuis le bord antérieur, et
l'angle (γ1) entre les éléments de tige (22, 23) est compris entre 30° et 120° sur le côté postérieur et l'angle (γ2) entre les éléments de paroi de tige (24, 25) est compris entre 90° et 180° sur le côté antérieur.

10. Plateau tibial (20) selon au moins l'une des revendications précédentes, dans lequel la structure de tige du plateau tibial (21) comprend au moins une structure de retenue de ciment (28), en particulier un trou, une rainure et/ou un évidement sans aucune contre-dépouille, optionnellement dans lequel l'au moins une structure de retenue de ciment (1) (20) est située à l'intérieur des deux tiers verticaux de la structure de tige du plateau tibial (21), mesurés depuis la surface distale (D) dans la direction distale (DD), en particulier à l'intérieur du premier tiers.

11. Plateau tibial (20) selon au moins l'une des revendications précédentes, dans lequel au moins un élément de fixation antérieur (2), en particulier exactement un élément de fixation antérieur (2), est positionné sur le côté antérieur de la surface proximale (P), le côté postérieur de l'au moins un élément de fixation antérieur (2) comprenant une paroi (4) qui, dans un plan parallèle à la surface proximale (P), est perpendiculaire au plan médian (M) de la surface proximale (P),
optionnellement dans lequel l'au moins un élément de fixation antérieur (2) comprend au moins un élément à complémentarité de forme (5), en particulier au moins une cavité dans la paroi postérieure (4) de l'au moins un élément de fixation antérieur (2) et/ou au moins un canal à travers l'au moins un élément de fixation antérieur (2), pour un élément à complémentarité de forme correspondant (31) de l'insert tibial (30), l'au moins un élément à complémentarité de forme (5) de l'au moins un élément de fixation antérieur (2) étant en particulier positionné dans une région dans laquelle le transfert de charge est inférieur à 70 %, en particulier 50 %, du transfert de charge maximal,
optionnellement dans lequel l'au moins un élément de fixation antérieur (2) comprend au moins une surface de guidage (11) pour un insert tibial (30), en particulier la surface de guidage (11) présentant une partie courbe et/ou une partie plane, dans une direction proximale de l'au moins un élément à complémentarité de forme (5),
optionnellement dans lequel l'au moins une surface de guidage (11) comprend une partie plane ou est un plan qui est incliné par rapport au plan horizontal selon un angle compris dans la plage entre 5 et 45°, en particulier 30°.

12. Plateau tibial (20) selon au moins l'une des revendications précédentes, avec une surface proximale (P), la surface proximale (P) comprenant au moins deux, en particulier exactement deux, éléments de fixation centraux (1) pour permettre une connexion avec l'insert tibial (30) en tant qu'autre partie d'une articulation artificielle du genou, dans lequel les au moins deux éléments de fixation centraux (1) sont des saillies depuis la surface proximale (P) dans la direction proximale et les au moins deux éléments de fixation centraux (1) sont positionnés symétriquement et/ou parallèlement à un plan médian (M) de la surface proximale (P), le plan médian (M) étant perpendiculaire à la surface proximale (P) et coupant la surface proximale (P) au milieu entre les deux points les plus latéraux (L1, L2) de la surface proximale (P).

13. Plateau tibial (20) selon au moins l'une des revendications précédentes, dans lequel les au moins deux éléments de fixation centraux (1) comprennent un évidement d'élément de fixation central (12).

14. Plateau tibial (20) selon au moins l'une des revendications précédentes, dans lequel au moins un, en particulier exactement un, élément de fixation postérieur (3) est positionné symétriquement par rapport au plan médian (M) de la surface proximale (P), comprenant une paroi postérieure (7) affleurant avec le bord postérieur (8) du plateau tibial (20) et/ou comprenant une paroi antérieure (9) qui est essentiellement positionnée parallèlement à la paroi postérieure (4) de l'au moins un élément de fixation antérieur (2),
optionnellement dans lequel les parois latérales (10) et/ou la paroi antérieure (9) de l'au moins un élément postérieur (3) sont inclinées selon un angle compris entre 0° et 45°, en particulier entre 0 et 30°, plus particulièrement selon un angle de 10°, par rapport à un plan qui est perpendiculaire au plan proximal (P) et/ou les parois latérales (10) et la paroi antérieure (9) de l'au moins un élément postérieur (3) comprennent une contre-dépouille.

15. Plateau tibial (20) selon au moins l'une des revendications précédentes, dans lequel les au moins deux éléments de fixation centraux (1) sont orientés symétriquement et/ou parallèlement au plan médian (M) ou les au moins deux éléments de fixation (1) sont orientés asymétriquement par rapport au plan médian (M).

16. Plateau tibial (20) selon au moins l'une des revendications précédentes, dans lequel il est fabriqué entièrement ou en partie en céramique, en un matériau polymère ou en métal.

17. Articulation artificielle du genou (100) comprenant un plateau tibial (20) selon au moins l'une des revendications 1 à 16 et un insert tibial (30), optionnellement dans lequel il existe au moins une région d'ajustement serré (38) entre l'élément de fixation postérieur (3) et l'insert tibial (20), en particulier créée par un désaccord angulaire entre deux parois.
